# EUROPEAN PATENT APPLICATION

(11) **EP 3 912 774 A1**
(43) Date of publication of application: **24.11.2021**
(21) Application number: 20315249.1
(22) Date of filing: 19.05.2020
(51) Int. Cl.: B26B 21/22, B26B 21/40

(54) **RAZOR CARTRIDGE WITH MAGNETIC ELEMENT**

(71) Applicant: Société BIC, 92110 Clichy (FR); BIC-Violex S.A., 145 69 Anixi, Attiki (GR)
(72) Inventor: Wright, Christopher John, London E15 4AD (GB); Duffy, David Michael, 8003 Zurich (CH); Zanidaki, Maria-Thiresia, 145 69 Anixi, Attiki (GR); Vlachos, Georgios, 145 69 Anixi, Attiki (GR)
(74) Representative: Peterreins Schley

(57) **Abstract**

The present invention relates to a razor cartridge 20 which comprises a frame 21 and one or more cutting members 28a-d. The frame 21 has a leading longitudinal member 24 and a trailing longitudinal member 25. The one or more cutting members 28a-d are arranged between the leading longitudinal member 24 and the trailing longitudinal member 25. At least one magnetic element 10 is configured and arranged to apply a magnetic field in a magnetic shaving aid comprising a magnetic surfactant and being applied to a skin surface K so as to elicit an induced change in the magnetic shaving aid between a pre-shave state and a during-shave state.

## Description

### Technical Field

The aspects described in the following disclosure relate to a razor cartridge, a razor, a hand-held dispenser and a razor kit for use with a shaving aid.

### Background

Razors (also known as safety razors) have a razor cartridge that is permanently or removably attached to a razor handle which, in use, is oriented in shaving direction. Razor cartridges typically comprise one or more cutting members, each including a blade, mounted perpendicular to the shaving direction. Razor cartridges are also typically (but not necessarily) provided with a guard (at a leading longitudinal side of the razor cartridge in the shaving direction) and a cap (at a trailing longitudinal side of the razor cartridge in the shaving direction). In use, a user holds the razor handle in the shaving direction and brings the razor cartridge into contact with a portion of skin defining a shaving plane.

Typically, the shaving plane is defined as the tangential line intersecting the first and second skin contact points of, for example, cutting edges of the razor cartridge. More simply, the shaving plane may be approximated as a line between the highest points on the skin-contacting surface of a razor cartridge - for example, the flat plane between the top of a guard and the top of a cap of the razor cartridge. During a shaving operation, movement of the razor handle causes the blades of the razor cartridge to be moved across the shaving plane in the shaving direction, enabling the blades to remove unwanted hair.

However, in such a shaving operation and due to the direct contact of the blades to the skin and resulting friction, discomfort may be present and skin irritations, nicks and cuts may occur. This may be the result of the blades contacting the skin and a corresponding abrasion or cutting of outer skin layers. Furthermore, the quality of the shave may be reduced as hairs may be missed which lie against or in close proximity to the skin. This leads to an inefficient shaving process that requires multiple strokes and may further cause irritation, nicks and cuts, since the user needs to repeatedly shave a skin region. In order to reduce skin irritations, discomfort and skin cuts during shaving operations, various approaches have been pursued in the state of the art. Some razors are known in the state of the art that improve gliding characteristics over the skin during a shaving operation using an additional shaving aid (e.g. a shaving cream or shaving foam) which is applied to the skin and acts as some kind of safety barrier between the delicate skin and the sharp razor blades. Thereby, the friction between the razor blades and the skin surface and skin irritations can be reduced.

However, despite the many advantages, using a shaving aid involves several drawbacks. On the one hand, if the shaving aid is too stiff, this may result in an increased resistance to the movement of the razor cartridge during the shaving operation. A large stiffness of the shaving aid may also lead to clogging of the razor cartridge and may impede the rinsability of the razor during and after the shaving operation. Furthermore, the shaving aid, hair and debris may be stick among the blades which can dull the razor. These factors can reduce the quality of the shave. As the shaving aid is present between the skin and the razor cartridge during shaving, the razor blades can be distanced farther away from the skin which intensifies the problem that hair strands may be cut too high or that hairs may be completely missed. On the other hand, if the shaving aid is made less stiff and more fluid, the shaving aid may flow from the skin before the shaving operation begins and may not sufficiently protect the skin from irritations. Thus, there is a trade-off between these two shaving aid configurations. Still, using a razor with a shaving aid of any of these configurations does not factor the difficulty of cutting hairs which lie against or in close proximity to the skin which might require multiple strokes to achieve an acceptable shaving result.

Accordingly, the present invention aims to provide a razor cartridge through which the shaving performance of a razor during a shaving operation is further improved. In particular, the present invention aims at enhancing the quality of the shaving result and at reducing irritation that may occur by a movement of the razor cartridge.

### Summary

The present invention relates to a razor cartridge according to claim 1, a razor according to claim 5, a hand-held dispenser according to claim 8 and a method for avoiding skin irritation improving the shave efficiency according to claim 15.

The razor cartridge according to the invention comprises a frame having a leading longitudinal member and a trailing longitudinal member, and one or more cutting members arranged between the leading longitudinal member and the trailing longitudinal member. The razor cartridge includes at least one magnetic element. The magnetic element is configured and arranged to apply a magnetic field in a magnetic shaving aid comprising a magnetic surfactant and being applied to a skin surface so as to elicit an induced change in the magnetic shaving aid between a pre-shave state and a during-shave state.

The razor according to the invention comprises a razor handle, a razor cartridge which is coupled to the razor handle. Furthermore, the razor includes at least one magnetic element. The magnetic element is configured and arranged to apply a magnetic field in a magnetic shaving aid comprising a magnetic surfactant and being applied to a skin surface so as to elicit an induced change in the magnetic shaving aid between a pre-shave state and a during-shave state. The magnetic element may be arranged at the razor handle or at the razor cartridge. If the razor comprises more than one magnetic element, one magnetic element may be arranged at the razor handle and one magnetic element may be arranged at the razor cartridge. Alternatively or additionally, two or more magnets may be arranged at the razor handle or at the razor cartridge. Additionally, one or more magnetic elements may be arranged at may be arranged at the razor handle or at the razor cartridge.

The hand-held dispenser according to the invention comprises a container and a manually operable dispensing means. The container contains a magnetic shaving aid composition comprising water and one or more surfactants comprising at least one magnetic surfactant. When operated, the manually operable dispensing means dispenses a portion of the magnetic shaving aid composition in response to positive pressure in the container. The positive pressure is:
a) build up by the user when operating the manually operable dispensing means or
b) present in the container due to the presence of a propellant within the magnetic shaving aid composition.

The razor kit according to the invention comprises a hand-held dispenser and a razor as described above.

The method for for avoiding skin irritation and improving the shave efficiency during a shaving operation by a user with a razor in combination with a magnetic shaving aid according to the invention comprises the steps of:
a) providing a razor having a razor cartridge and at least one magnetic element;
b) providing a magnetic shaving aid consisting of a magnetic shaving aid composition comprising a magnetic surfactant in a pre-shave state;
c) applying the magnetic shaving aid to a skin surface;
d) performing a shaving operation with the razor on the skin surface;
e) inducing a change in the magnetic shaving aid by applying a magnetic field in the magnetic shaving aid to bring the magnetic shaving aid from the pre-shave state to a during-shave state during performing the shaving operation.

It has been discovered that a trade-off between the two shaving aid configurations of a stiffer and a more liquid shaving aid can be overcome by eliciting an induced change in the shaving aid during a shaving operation. This induced change enables the shaving aid to assume different states with different characteristics optimized for different stages involved in a shaving operation. More specifically, these different states include a first state optimized for pre-shave, i.e. a "pre-shave state" and a second state optimized for during-shave, i.e. a "during shave state" which can lead to both - an improved shaving performance and an improved shaving comfort, i.e. to reduced skin irritations.

In the pre-shave state, a sufficient shaving aid stability (also called shaving aid strength) can be provided which reduces the risk that the shaving aid may flow away from the skin before the shaving operation begins. Thereby, the shaving aid can sufficiently protect the skin from irritations and may also have a thermally insulating effect for the skin.

In the during-shave state, improved lubrication properties between the skin surface and skin contacting elements of the razor cartridge can be provided which reduces friction and skin irritation. Furthermore, the shaving aid can be guided away from the cutting members after cutting hair which prevents clogging of the razor cartridge and facilitates clearance of the shaving aid including hair and debris during rinsing. Additionally, hairs (in particular hairs which lie against or in close proximity to the skin) can be oriented and lifted up from the skin surface towards the cutting members which enables removing more hairs with one stroke and cutting hairs at a lower cutting point, i.e. closer to the skin surface, resulting in an improved and more efficient shaving performance in some situations. In other words, more hairs can be brought above the shaving plane in order to be removed by the cutting members.

The above described advantageous effects are achieved by the specifically configured shaving device (razor cartridge and/or razor) in combination with the specifically configured shaving aid which is provided by the hand-held dispenser according to the present invention. On the one hand, the razor (and/or razor cartridge) comprises at least one magnet which enables the above described effects. Thereby, the arrangement (e.g. at leading longitudinal member, at trailing longitudinal member, above/behind/before the cutting members and/or at a distal end of the razor handle) and the configuration (static magnet, actively controlled magnetic fields by using an electromagnet, magnetic field strengths) of the magnetic element is specifically chosen and adapted in order to elicit the induced change in the shaving aid. On the other hand, the shaving aid has specific properties so that a change can be induced by the magnetic element of the razor (or razor cartridge). In detail, the shaving aid is provided in the container of the hand-held dispenser and comprises at least one magnetic surfactant in its composition. Therefore, the shaving aid is also denoted magnetic shaving aid or magnetic shaving aid composition. Alternatively, the magnetic shaving aid composition can be described as a cosmetic composition comprising magnetic surfactant(s) and acting as shaving aid.

The presence of a surfactant having magnetic nature, i.e. a magnetic surfactant, results in the fact that the magnetic surfactant is the driving factor responsible for the interaction of the shaving aid with the magnetic forces applied by the magnetic elements. In contrast to mere magnetic particles in a solution (e.g. ferrofluids), magnetic surfactants inherently contain magnetic properties. Rather than those typical ferrofluids which comprise magnetic colloidal particles (10 nm) being dispersed in a carrier fluid, magnetic surfactants are molecular substances which are nano-particle free. That means in magnetic surfactants, the magnetic component is part of each magnetic surfactant molecule and remains firmly bound to the molecule and groups of molecules. Hence, the magnetic surfactants are themselves paramagnetic. This leads to the major advantage in that a magnetic surfactant allows physicochemical properties (hydrophobicity, electrical conductivity, melting point, etc.) to be controlled by external magnetic fields. Therefore, by application of a magnetic field in the shaving aid containing those magnetic surfactants influencing, e.g., micellar structuring and surface properties (e.g. reduction of surface tension) of the shaving aid is possible.

This makes it possible to provide a shaving aid composition with a high shaving aid strength in the pre-shave state whilst also having a lowered shaving aid strength in the during-shave state by eliciting the induced change through the application of magnetic fields in the shaving aid. That means, the surface tension can be reduced which can improve the lubrication properties and the rinsability in the during-shave state. Furthermore, various arrangements of the magnetic element(s) above the skin surface promotes straightening and lifting of hair from the skin surface towards the magnetic element(s) by attracting magnetic forces which are exerted on the magnetic surfactants in the during-shave state. Consequently, this may facilitate hair removal and may enhance the efficiency and quality of the shaving operation. The inherent amphiphilic property (i.e. hydrophilic and hydrophobic) of surfactants (also of magnetic surfactants) can ensure that the surfactants pull hair with them. As the magnetic shaving aid is homogenous, the magnetic shaving aid (at least portions of the magnetic shaving aid) can also be pulled, or at least be oriented, with the magnetic surfactant without being separated. For instance, this is particularly advantageous, when a magnetic element is arranged at the trailing longitudinal member, in particular at an upper side, such that the shaving aid including hair and debris can be collected at the trailing longitudinal member and be easily rinsed after the shaving operation.

Consequently, the disclosure according to the invention enables to perform a more efficient shaving operation with reduced skin irritation and increased shaving comfort including the elicitation of an induced change in the shaving aid to achieve versatile effects and conditions for optimally handling the different stages of the shaving operation.

In the following specification, the term "cutting member" means a component of a razor cartridge that, in use, contacts the skin of a user and cuts protruding hairs. A cutting member can mean at least a razor blade having a blade with a cutting edge glued, or laser welded, to a separate bent support member. The bent support member is fitted into a cutting member support slot in-between two opposed cutting member guides, such as protrusions from a transverse frame member of the razor cartridge. The blade can be attached to the face of the bent support member that faces towards a user of the razor cartridge, in use. Alternatively, the blade can be attached to the face of the bent support member that faces away from a user of the razor cartridge, in use. In this latter case, each cutting member has two contact points with the skin of the user (the blade edge, and the distal end of the bent support member), to thus reduce pressure on the user's skin. Alternatively, the cutting member may be a "bent blade". This is an integrally formed cutting member comprising a radiused bend, and a cutting edge formed at a distal end of the radiused bend.

A "group of cutting members" may consist of the same type of cutting members, or may comprise at least one bent blade, or another type of blade for example.

In the following specification, the term "leading" means the side of the razor cartridge that contacts a portion of a user's skin first, in normal use.

In the following specification, the term "trailing" means the side of the razor cartridge that contacts a portion of a user's skin last, in normal use.

In the following specification, the local term "before" means a relative position with respect to the razor cartridge that is closer to the leading side, in normal use.

In the following specification, the local term "behind" means a relative position with respect to the razor cartridge that is closer to the trailing side, in normal use.

In the following specification, the term "above" means a relative position with respect to the razor cartridge that is farther away from the skin surface, in normal use. Analogously the term "upper" means a relative position with respect to the razor cartridge that is farther away from the skin surface, in normal use.

In the following specification, the local term "below" means a relative position with respect to the razor cartridge that is closer to the skin surface, in normal use. Analogously the term "lower" means a relative position with respect to the razor cartridge that is closer to the skin surface, in normal use.

In the following specification, the term "wt.-%" describes percentages by weight calculated with respect to the total weight of the magnetic shaving aid composition. Unless otherwise specified, all percentages and amounts expressed herein and elsewhere in the specification should be understood to refer to wt.-%.

In the following specification, the term "and mixtures thereof" is be understood as any mixture of the respective components regarding amounts by weight but also regarding the selection of one or more components. Alternatively, it could be described as selected from any of the components and any mixture thereof.

Additional details and features of the invention are described with reference to the drawings as follows.

### Description of the Drawings

Other characteristics will be apparent from the accompanying drawings, which form a part of this disclosure. The drawings are intended to further explain the present disclosure and to enable a person skilled in the art to practice it. However, the drawings are intended as nonlimiting examples. Common reference numerals on different figures indicate like or similar features.
- **FIG. 1a**: shows a perspective view of a razor including a magnetic element arranged at the razor cartridge;
- **FIG. 1b**: shows a perspective view of a razor including a magnetic element arranged at the razor handle;
- **FIG. 1c**: shows a perspective view of a razor including two magnetic elements and a comb arranged at the razor cartridge;
- **FIG. 2**: is a perspective partial exploded view of a razor cartridge.
- **FIG. 3**: is a schematic cutaway side view of a razor cartridge taken from the embodiment of FIG. 2 along axis P-Q
- **FIGs. 4a-4c**: are schematic side views of razor cartridges comprising one or more magnetic elements;
- **FIGs. 5a-5b**: show two different embodiments of a hand-held dispenser including the magnetic shaving aid composition;

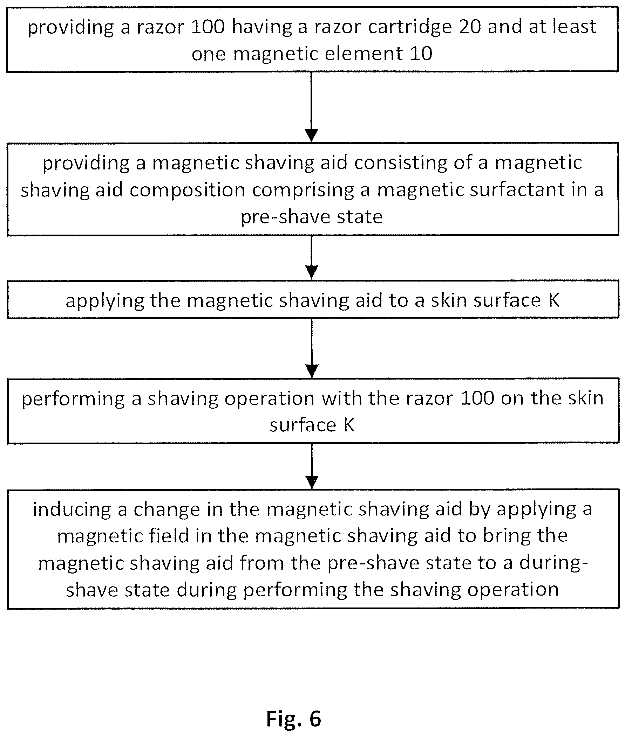

- **FIG. 6**: shows the method steps of a method for avoiding skin irritation and improving the shave efficiency.

### Detailed Description

Embodiments of the razor device (i.e. razor cartridge and razor), the hand-held dispenser, the razor kit and the method according to the invention will be described in reference to the drawings as follows.

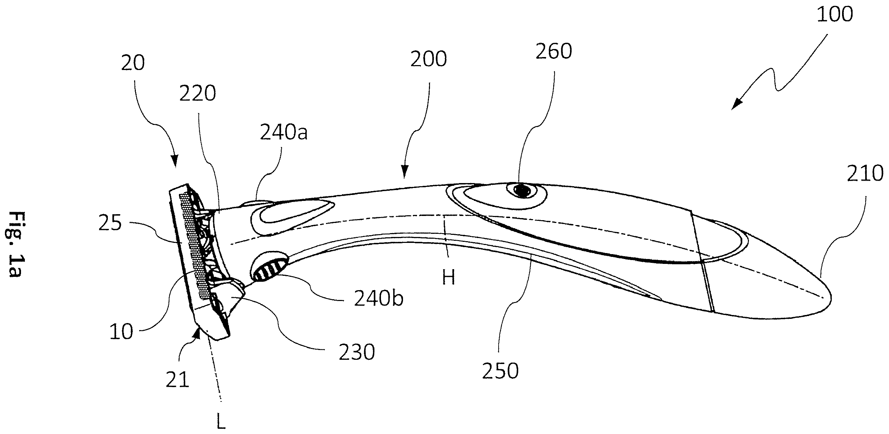

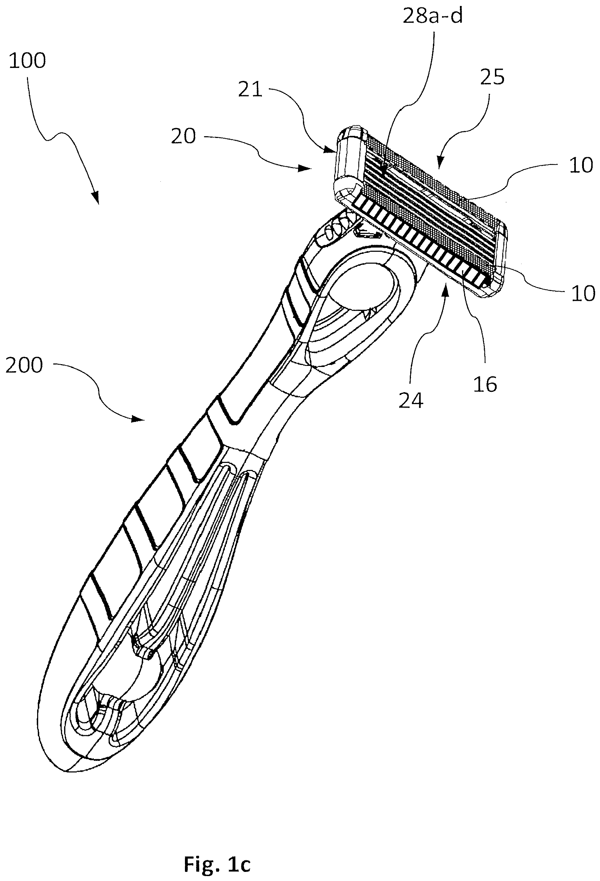

**Figs. 1a-1c** are perspective views of a razor 100. The razor 100 comprises a razor handle 200, a razor cartridge 20 and one or more magnetic elements 10. It should be noted that the razor 100 comprising the razor handle 200, the razor cartridge 20 and the magnetic element(s) 10 can be any wet shaving razor known in the state of the art including shaving blades, wherein hairs are removed due to a movement, in particular due to shaving strokes in shaving direction S, by a user on the skin surface K (also denoted skin or user's skin). Alternatively, the razor 100 comprising the razor handle 200, the razor cartridge 20 and the magnetic element(s) 10 can be any electrically operated razor (or dry razor) as known in the state of the art, wherein the razor 100 comprises a rotating or oscillating blade, powered by an electric module (e.g. a battery). The razor cartridge 20 comprises a frame 21 with a leading longitudinal member 24 and a trailing longitudinal member 25 (see **Fig. 1c**). One or more cutting members 28a-d (in the present example four) are arranged between the leading longitudinal member 24 and the trailing longitudinal member 25.

The razor handle 200 extends in a handle direction H between a proximal portion 210 and a distal portion 220 of the razor handle 200 (see **Figs. 1a-1b**). The razor cartridge 20 is mounted at the distal portion 220 of the razor handle 200. The mounting of the razor cartridge 20 to the distal portion 220 of the razor handle 200 in the illustration is, in an embodiment, via a coupling 230 (also denoted pivotable bearing member), in an example, a pivotable bearing member, enabling a frame of reference of the razor handle 200 to vary relative to a frame 21 of reference of the razor cartridge 20. This enables the angle of the razor cartridge 20 against the skin of a user to vary and adapt to changes during use.

In particular, the razor cartridge 20 pivots relative to the razor handle 200 about the longitudinal axis L of the razor cartridge 20, in use. The pivoting enables the user to adapt to contours of the body, for example. The longitudinal axis L of the razor cartridge 20 is substantially perpendicular to the shaving direction S along the razor handle 200. Another example of a connection mechanism for connecting the razor cartridge 20 to the razor handle 200 is discussed in WO2006/027018 A1. Another example is a razor cartridge 20 that may pivot relative to a second pivot axis (a rocking axis), substantially perpendicular to axis L.

In embodiments, the pivotable bearing member 230 may be omitted (not illustrated) and the handle 200 may be provided as an integrally connected part of the support of the razor cartridge 20. In an example, the pivotable bearing member 230 may further comprise, or be replaced by, a release mechanism 240a, 240b, enabling rapid release of an exhausted razor cartridge 20 from the razor handle 200.

In an embodiment, the razor handle 200 and the support of the razor cartridge 20 are integrally formed with a pivotable bearing member (not illustrated) such as a resilient plastic spring member.

In an embodiment, the frame 21 of the razor cartridge 20 is connectable to the razor handle 200 of the razor 100 either integrally, or by a connection mechanism such as the pivotable bearing members 230 or by an interconnecting member (not shown). Although not illustrated, the pivotable bearing member 230, in an embodiment, may be provided on the side of the razor cartridge 20 and may be configured to connect to a pivotable handle. The pivotable bearing member 230, in an example, comprises two or more shell bearings configured to connect to a pivotable bearing member of the razor handle 200.

In an embodiment, the razor handle 200 is provided with a handle grip 250 formed of a rubber, or rubber-like material to improve gripping friction.

According to various aspects of the invention, one or more magnetic elements 10 can be arranged at different locations on or in the razor 100, i.e. at different locations at the razor cartridge and/or at the razor handle 200. In detail, the arrangement and the configuration of the magnetic element(s) is specifically chosen and adapted to apply a magnetic field in a magnetic shaving aid which comprises a magnetic surfactant and which is applied to the skin surface K. This is done in such a manner so as to elicit an induced change in the magnetic shaving aid between the pre-shave state and the during-shave state. In other words, the induced change elicited by the one or more magnetic elements 10 enables achieving versatile effects and conditions for optimally handling the different stages of the shaving operation as described further above. These effects and conditions can involve physical movement of the magnetic shaving aid and manipulation of bulk properties of the magnetic shaving aid. In particular, magnetic surfactants are affected on the molecular scale, where the magnetic field alters their surfactant properties, rather than just at the macro scale inducing movement. This can change surface tension, alter foaming behavior etc. The key distinction therefore is the modulation of molecular surfactant properties.

As explained above in further detail, physical movement due to attracting magnetic forces can include attraction of the shaving aid including removed hair and debris towards the magnetic element(s) 10 but also lifting up of hairs to which the magnetic shaving aid (i.e. the magnetic surfactant) is attached to. This results in a lower cutting point and less hairs lying against the skin surface K which consequently facilitates hair removal and enhances the efficiency and quality of the shaving operation. Also clogging of the razor cartridge 20 during a shaving operation can be prevented as the magnetic shaving aid including removed hair and debris won't dispense on cutting members 28a-d of the razor cartridge 20 but will be drawn towards magnetic element(s) 10 which further improves the shave quality and comfort. Manipulation of bulk properties mainly involves the reduction of the surface tension of the magnetic shaving aid under the influence of magnetic forces. This enhances gliding properties and reduced friction which leads to less skin irritations and an easier rinsability.

The term "different stages of the shaving operation" is to be understood in that it can also include preparing and follow up operations of the shaving operation, i.e. all stages from providing a shaving aid on the a user's skin to rinsing the shaving device (i.e. operations which are commonly conducted by a user). The term "pre-shave state" describes certain properties and conditions which are advantageous before starting the shaving operation, however this term should not be understood limiting to only the stage before starting the shaving operation. The pre-shave state is usually present when no or only a magnetic field having insufficient magnetic field strength affects the magnetic shaving aid. Analogously, the term "during-shave state" describes certain properties and conditions which are advantageous during the shaving operation, however this term should not be understood limiting to the stages during the shaving operation. The during-shave state is usually present when a magnetic field, i.e. a magnetic field with a sufficient field strength, affects the magnetic shaving aid.

In the following various arrangements/configurations of magnetic element(s) will be explained, whereby it is mentioned that only some of these arrangements/configurations are illustrated in the drawings.

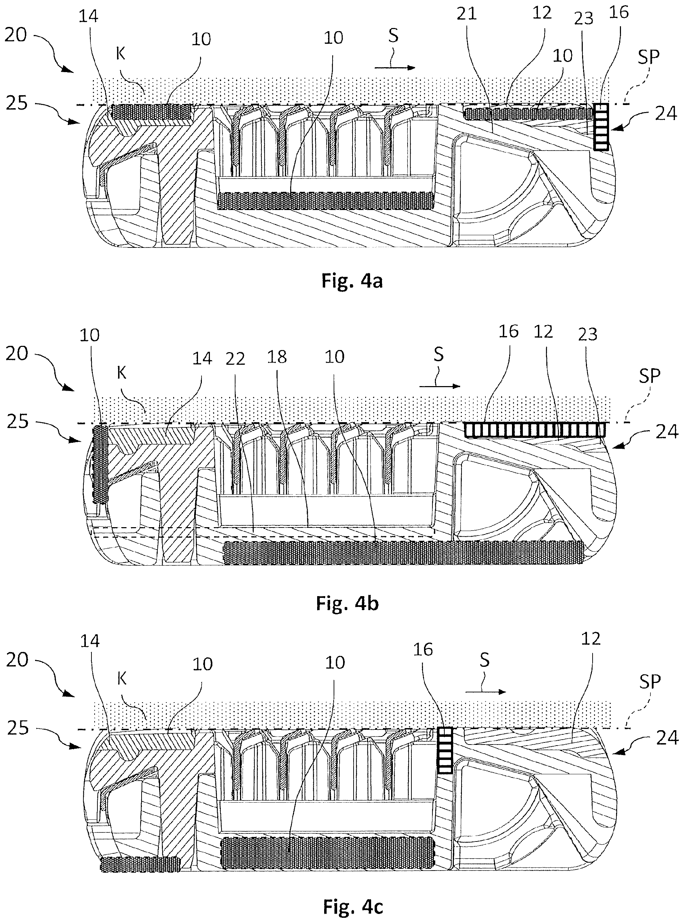

With regard to **Fig. 1a****,** an exemplary embodiment is illustrated, wherein the magnetic element 10 is arranged at the razor cartridge 20. The magnetic element 10 is thereby arranged at the trailing longitudinal member 25 of the razor cartridge 20. More specifically, the magnetic element 10 is arranged at an upper portion of the trailing longitudinal member 25. This specific arrangement results in the advantage that the magnetic shaving aid including removed hairs and debris can be easier collected behind the cutting members 28a-d after cutting hair. This prevents clogging of the razor cartridge 20 and facilitates clearance of the magnetic shaving aid including removed hair and debris during rinsing. In another aspect, the magnetic element 10 may be arranged elsewhere at the trailing longitudinal member 25 or elsewhere at the razor cartridge 20 (see, e.g. explanations further below with regard to **Figs. 4a-4c**).

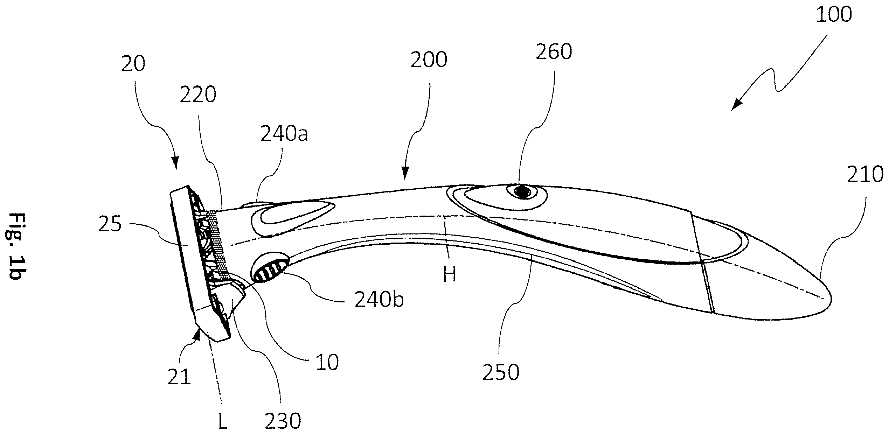

In a further aspect, the magnetic element 10 may additionally or alternatively be arranged at the razor handle 200 which is shown in **Fig. 1b****.** In this embodiment, the magnetic element 10 is arranged at, in particular attached to, a distal portion 220 of the razor handle 200. The magnetic element 10 is attached to the distal portion 220 directly adjacent to the razor cartridge 20, in particular, above the cutting members 28a-d of the razor cartridge 20. Thereby, it can be ensured that the magnetic field strengths provided by the magnetic element 10 are sufficient in order to achieve the induced change in the magnetic shaving aid. Another advantage of this embodiment is that hair which is to be removed will be lifted up towards the cutting members 28a-d as the magnetic element 10 is arranged directly above the cutting members 28a-d. Another advantageous effect of this embodiment is that the magnetic element 10 is reusable when an exhausted razor cartridge 20 has to be replaced. This safes costs and protects the environment.

Although in **Figs. 1a** **and** **1b** only one magnetic element 10 is shown, respectively, the razor handle 200 and/or the razor cartridge 20 may comprise more than one, for instance two, three or four magnetic elements 10. In this regard, **Fig. 1c** depicts another embodiment, wherein the razor cartridge 20 comprises two magnetic elements 10. A first magnetic element 10 is arranged at the leading longitudinal member 24 and a second magnetic element 10 is arranged at the trailing longitudinal member 25. In particular, the first magnetic element 10 is arranged at a lower side of the leading longitudinal member 24 (i.e. at a side facing towards the skin surface K during use). The second magnetic element 10 is arranged at a lower side of the trailing longitudinal member 25 (i.e. at a side facing towards the skin surface K during use). Thereby, the first magnetic element 10 can primarily lift up hairs before the cutting members 28a-d. The second magnetic element 10 can primarily collect used magnetic shaving aid including removed hairs and debris behind the cutting members 28a-d which facilitates hair removal and improves the shave quality and comfort. In another embodiment (not shown), the razor cartridge 20 may further comprise a third magnetic element 10 which is, for instance arranged above the cutting members 28a-d. In another aspect, the first magnetic element 10 and/or the second magnetic element 10 and/or one or more additional magnetic elements 10 may be arranged elsewhere at the leading and trailing longitudinal members 24, 25 or elsewhere at the razor cartridge 20.

In other embodiments (not shown), both - the razor cartridge 20 and the razor handle 200 may comprise one or more magnetic elements 10.For instance, the razor cartridge 20 may comprise two or three magnetic elements 10. For instance, the razor handle 200 may comprise two or three magnetic elements 10. For instance, in one aspect, a first magnetic element 10 may be arranged at the razor cartridge 20 as described with respect to **Fig. 1a** and a second magnetic element 10 may be arranged at the razor handle 200 as described with respect to Fig. **1b****.** Additionally a third magnetic element 10 may be arranged at the razor cartridge 20 and/or at the razor handle 200. Additionally a fourth magnetic element 10 may be arranged at the razor cartridge 20 and/or at the razor handle 200. Additionally a fifth magnetic element 10 may be arranged at the razor cartridge 20 and/or at the razor handle 200 and so on. In this regard, for instance, a first magnetic element 10 and a second magnetic element 10 may be arranged at the razor cartridge 20 as described with respect to **Fig. 1c** and a third magnetic element 10 may be arranged at the razor handle 200 as described with respect to **Fig. 1b****.** In embodiments, either the razor cartridge 20 may comprise magnetic element 10 or the razor handle may comprise magnetic element 10, or both.

In another aspect, the magnetic element(s) 10 are configured to provide magnetic field strengths of 0.01-1 Tesla at a distance of 0.0 cm to 2.0 cm, specifically 0.0 cm to 1.5 cm, and most specifically 0.0 cm to 1.0 cm from a surface of the magnetic element 10, in particular a surface of the magnetic element 10 which is closest to the skin surface K. The stronger the magnetic field strength and the closer the magnetic element 10 is to the magnetic shaving aid, the stronger will be the induced change, in particular the reduction in surface tension of the magnetic shaving aid and the induction of magnetic pulling forces which are exerted on the magnetic shaving aid by applying the magnetic field to the magnetic surfactant. Particularly the magnetic element(s) are configured to provide a magnetic field strength of 0.4 Tesla in the magnetic shaving aid. However, if the magnetic field strengths are too high, the magnetic pulling forces exceed the adhering strength of the magnetic shaving aid (i.e. the magnetic surfactant) on the hairs which are to be removed such that the magnetic shaving aid is lifted up at least partly from the skin surface K. This would be detrimental during the shaving operation but might be advantageous after the shaving operation in order to clear the cutting members from used shaving aid including removed hairs and debris. Thus, one or more magnetic elements 10 with increased magnetic field strengths, i.e. larger than 1 Tesla, may be provided which can be activated after a cutting stroke or in between two cutting strokes in order to elicit an induced change in used shaving aid including removed hairs and debris.

The magnetic element 10 may be configured to provide a static magnetic field or an actively controlled magnetic field. To provide a static magnetic field the magnetic element 10 can be configured as a solid state magnet. To provide an actively controlled magnetic field the magnetic element 10 can be configured as an electromagnet. In some aspects, combinations of static magnetic fields and actively controlled magnetic field may be used. That means, more than one magnetic element 10 may be provided whereby at least one magnetic element 10 is a solid state magnet and at least another magnetic element 10 is an electromagnet. For instance, in the above described embodiment wherein a first magnetic element 10 is arranged at the razor cartridge 20 and a second magnetic element 10 is arranged at the razor handle 200, the first magnetic element 10 may be a solid state magnet and second magnetic element 10 may be an electromagnet. This has the cost advantage that the more expensive electromagnet can be reused when an exhausted razor cartridge 20 has to be replaced. However, it should be understood that any one, some or all magnetic elements 10 may be electromagnets. In another aspect, the magnetic element(s) (one some or all) may be coated in a low friction surface coating in order to enable easy cleaning.

According to an aspect, the razor 100 comprises a switch 260. The switch 260 is configured and arranged to control the magnetic element 10. In the embodiments of **Figs. 1a-****1b,** the switch 260 is arranged at the razor handle 200. Alternatively, the switch 260 may be arranged at the razor cartridge 20 (not shown). In some embodiments (not shown), two switches 260 may be provided, a first switch 260 which is arranged at the razor handle 200 and a second switch 260 which is arranged at the razor cartridge 20. In some embodiments (not shown), more than one switch 260 may be arranged at the razor handle 200 and/or at the razor cartridge 20. By the provision of the switch the induced change is more accurately controllable (i.e. by adjusting the magnetic fields of one or more magnetic elements 10 at different locations on the razor handle 200 and/or on the razor cartridge 20) and reversible (i.e. from during-shave state to pre-shave state by deactivating the magnetic fields of one or more magnetic elements 10 at different locations on the razor handle 200 and/or on the razor cartridge 20) even during the shaving operation. This leads to the effect that the different stages of the shaving operation can be handled more appropriately.

According to an aspect, the razor 100 comprises a switch structure (not shown) which is attached to or integrated into the razor handle 200. Alternatively, the switch structure may be attached to or integrated into the razor cartridge 20. The switch structure is coupled to or part of the switch 260 and provides the operable coupling means to control the magnetic element 10 with the switch 260 (only the operating means of the switch 260, i.e. an operating button of the switch 260 is visible in **Figs. 1a-1b**). The switch structure is configured to be coupled to a solid state magnet and/or to an electromagnet. In other words, the switch structure is configured to operably couple the switch 260 to the magnetic element 10. When the switch structure is to be coupled to a magnetic element 10 which is an electromagnet it may comprise, inter alia, a control unit, electrical control lines which connect the control unit with the switch 260 and with the magnetic element 10, and a power supply such as a battery or a plug (not illustrated, but known in the art) such that the switch 260 can control the magnetic element 10. When the switch structure is to be coupled to a magnetic element 10 which is a solid state magnet it is configured as a mechanical switch structure or permanent magnet switch which is known in the art.

According to an aspect, the switch 260, i.e. the operating button of the switch 20, can for instance be movable in a direction vertical to the handle direction H, wherein a user applies a force on the switch 260 in vertical direction in order to activate the magnetic element(s) 10. Additionally or alternatively, the switch 260 may be movable in a direction parallel to the handle direction H, wherein a user applies a force on the switch 260 in parallel direction. Thereby, the switch 260 may be movable between at least two distinct positions (vertical and/or parallel), wherein each distinct position leads to a distinct behaviour of the magnetic element(s) 10, for instance, a first distinct position which turns the magnetic element(s) 10 on and a second distinct position which turns the magnetic element(s) 10 off. Additionally, one or more intermediate distinct positions are possible whereby magnetic fields with intermediate magnetic field strengths between zero and a maximum magnetic field strength can be provided by the magnetic element(s) 10. If more than one magnetic element 10 is present some or all may be controlled together with one switch 260. Additionally or alternatively, for one switch 260 may be provided for each or at least some of the magnetic elements 10 or for groups of magnetic elements 10. Additionally or alternatively, different distinct positions of the switch 260 may activate different magnetic elements 10. For instance, in a first neutral position the switch 260 may not activate any of the magnetic elements 10. In a second position adjacent to the first position along the handle direction H towards the distal portion 220 of the razor handle 200, the switch 260 may activate a first magnetic element 10. In a third position adjacent to the first position along the handle direction H towards the proximal portion 210 of the razor handle 200, the switch 260 may activate a second magnetic element 10. Additionally distinct intermediate positions may be provided for varying the magnetic field strengths and/or for activating all magnetic elements 10 at the same time. Thereby, different magnetic elements 10 can be activated at different stages of the shaving operation. This provides the possibility to elicit an induced change in the magnetic shaving aid at different times, i.e. at different stages, and/or at different locations of the razor 100, in particular different locations of the razor cartridge 20 which enhances the control accuracy of the induced change.

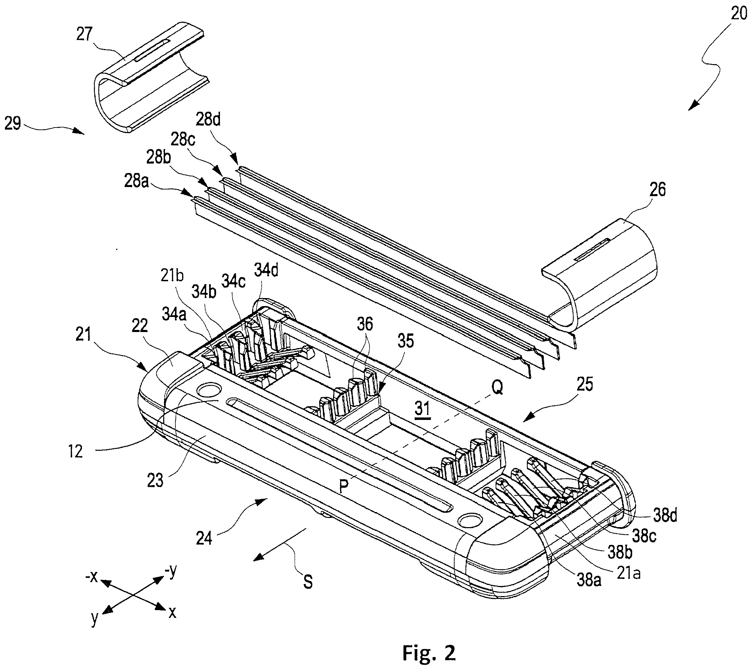

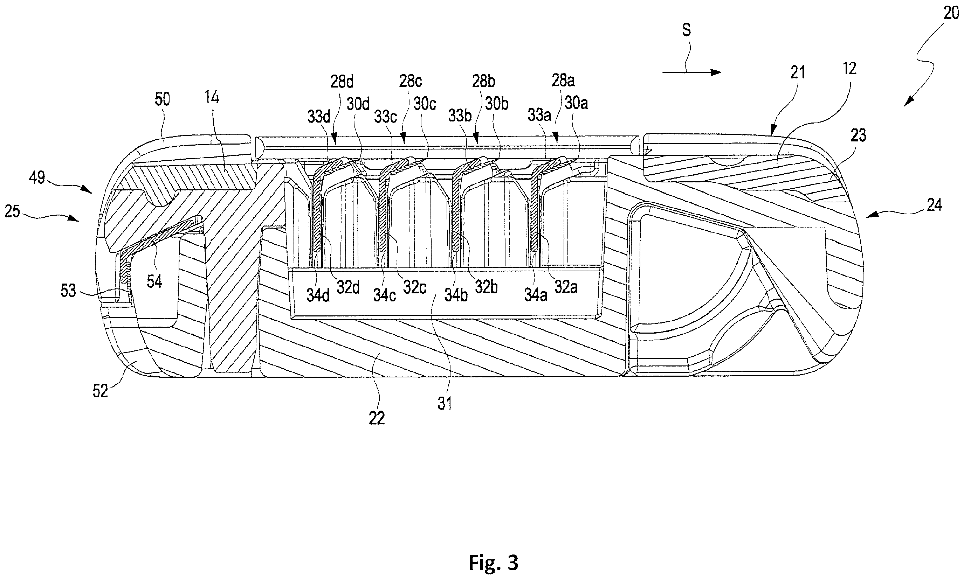

**Figs. 2** **and** **3** illustrate a perspective partial exploded view and a schematic cutaway side view of the razor cartridge 20 taken from the embodiment of FIG. 2 along axis P-Q according to a second aspect. "Partial exploded view" means that some minor components of the razor cartridge 20 have been omitted from the exploded view to aid clarity of the drawing.

As mentioned above, the razor cartridge 20 comprises a frame 21. The frame 21 comprises a leading longitudinal member 24 and a trailing longitudinal member 25 and at least one transverse frame member 35 disposed in between, and joining, the leading longitudinal member 24 and the trailing longitudinal member 25, in a transverse direction of the razor cartridge 20.

The at least one transverse frame member 35 comprises a plurality of cutting member guides 36 defining a plurality of cutting member support slots 34a-d, each cutting member support slot configured to accommodate a longitudinal cutting member 28a-d.

The shaving direction S is depicted in **Fig. 2** using arrow S. In use, the razor cartridge 20 contacts a shaving plane SP, and is translated by the user across the shaving plane SP in the direction of arrow S.

A frame 21 may be fabricated partially or completely of synthetic materials, such as plastic, resin, or elastomers. The frame 21 comprises a platform member 22. A guard member 23 is, in an example, provided as a substantially longitudinal edge of the razor cartridge 20. In use, the guard member 23 is the first portion of the razor cartridge 20 to contact uncut hairs, and it is thus located at a leading longitudinal member 24 of the razor cartridge 20. The side of the razor cartridge 20 opposite to the leading longitudinal member 24 of the razor cartridge 20 and opposite to the shaving direction S is the trailing longitudinal member 25 of the razor cartridge 20. The trailing longitudinal member 25 is thus the final portion of the razor cartridge 20 to contact the shaving plane SP, in use.

One or more skin contacting elements 12, 14 may be provided at the razor cartridge 20. In the embodiment according to **Figs. 2-3****,** the razor cartridge 20 comprises a first skin contacting element 12 and a second skin contacting element 14. The first skin contacting element 12 is arranged at the leading longitudinal member 24, in particular at a lower portion of the leading longitudinal member 24 which faces towards the skin surface K during use. The second skin contacting element 14 is arranged at the trailing longitudinal member 25, in particular at a lower portion of the trailing longitudinal member 25 which faces towards the skin surface K during use. The skin contacting elements 12, 14 are configured to contact a user's skin surface K during a shaving operation. The skin contacting elements 12, 14 of the razor cartridge 20 face and contact the skin surface (K) during a shaving operation. In the embodiment of Fig. 3, the first skin contacting element 12 and the guard member 23 are integrally formed as one part. In alternative embodiments, the first skin contacting element 12 and the guard member 23 may be separate parts.

It will be noted that the terms "leading longitudinal member 24" and "trailing longitudinal member 25" are used to denote specific locations on the razor cartridge 20, and do not imply or require the absence or presence of a particular feature. For example, a guard member 23 and/or a skin contacting element 12 may in one example be located at the side comprising the "leading longitudinal member 24", and a trimming blade 53 and/or a skin contacting element 14 may be located at the side comprising the "trailing longitudinal member 25" in another example, but it is not essential that these sides of the razor cartridge 20 comprise such features.

The guard member 23, in an example, comprises an elastomeric member (not shown in **Fig. 2****).** In an example, the elastomeric layer comprises one or more fins extending longitudinally in parallel to the guard member 23 and substantially perpendicularly to the shaving direction. One purpose of such an elastomeric layer is, for example, to tension the skin prior to cutting.

The razor cartridge 20 may, in embodiments, further comprise a cap member 29 at, or near to, the trailing longitudinal side 25 but this is not illustrated in the embodiment of **Fig. 2** as an aid to clarity.

As already described further above, the razor cartridge 20 comprises a group of cutting members 28a-d accommodated in a cutting member receiving section 31 of the frame 21. The group of cutting members 28a-d comprises a plurality of longitudinal cutting members 28a-d. In embodiments, each of the longitudinal cutting members 28a-d comprises a blade 33a-d having a cutting edge 30a-d. The group of cutting members 28a-d is disposed in the frame 21 longitudinally and transverse to the shaving direction S such that in use, the blades 33a-d of the cutting members 28a-d contact a shaving plane SP and cut hair present on the shaving plane SP as the razor cartridge 20 is moved across the shaving plane SP in the shaving direction S.

The razor cartridge 20 is provided with four cutting members 28a-d. In embodiments, the razor cartridge 20 can be provided with at least one cutting member 28. In particular, the razor cartridge 20 can be provided with one cutting member, two cutting members, three cutting members, four cutting members, five cutting members, six cutting members, seven cutting members or more cutting members.

The group of cutting members 28a-d defines a plurality of substantially parallel inter-blade spans. In conventional razor cartridges having blades above the support, with three or more blades, each inter-blade span is measured to be constant in a range of about 1.05 mm to 1.5 mm. The number of inter-blade spans is one fewer than the number of cutting members.

The frame 21 further comprises a first retainer 26 and a second retainer 27 configured to hold the cutting members 28a-d within razor cartridge 20 housing. The frame 21 further comprises first 21a and second 21b side portions. When the razor cartridge 20 is assembled, the first and second side portions 21a, 21b are configured to confine the longitudinal ends of the guard member 23, a cap member (if present, not shown in **Fig. 2****)** and the group of cutting members 28a-d. The first side retainer 26 and second retainer 27 may comprise, for example, plastic, an elastomer, or a metal material and furthermore may be of a different shape to that illustrated.

In an example, the cutting members 28a-d comprised in the group of cutting members 28a-d are disposed in the razor cartridge 20 such that two cutting edges 30a,b comprised, respectively, on the two foremost (nearest to the leading longitudinal member 24 of the razor cartridge 20) cutting members 28a,b of the group of cutting members 28a-d define a leading inter-blade span that is closest to the leading longitudinal side 24 of the razor cartridge 20 and that is greater than a trailing inter-blade span defined between the two cutting edges that are closest to the trailing longitudinal side 25 of the razor cartridge 20.

The razor cartridge 20 of **Fig. 2** comprises four resilient fingers 38a, 38b, 38c, 38d under the first retainer 26. The razor cartridge 20 comprises four resilient fingers under the second retainer 27 that are in transverse corresponding alignment with the four resilient fingers 38a, 38b, 38c, 38d under the first retainer 26.

In total, the eight resilient fingers each exert a bias force against respective cutting members 28a-d of the group of cutting members 28a-d in the direction of the shaving plane SP, such that the cutting members 28a-d of the group of cutting members 28a-d are in a rest position, when the razor cartridge 20 is assembled. In the rest position, the cutting edges 30 of the blades 33 of the cutting members 28a-d, bear against corresponding stop portions at each lateral end of the blades 33a-d near the first 26 and second 27 retainers, for example. In an example, the stop portions may be the first 26 and second 27 retainer.

Accordingly, the rest position of the cutting members 28a-d is well defined, enabling a high shaving precision. Of course, the illustrated biasing arrangement has many variations. For example, a further plurality of resilient fingers may be provided on one or more of the transverse frame members 35. In a simplified razor cartridge design (such as for low cost, disposable razors), the resilient fingers may be omitted. A skilled person will appreciate that the number of resilient fingers 38 to be provided is related to the number of cutting members 28a-d in the group of cutting members 28a-d, and that fewer or more than eight resilient fingers 38 can be provided.

In an example, each cutting member 28a-d in the group of cutting members 28a-d comprise a longitudinal blade support 32a-d. A longitudinal blade 33a-d is mounted on the respective blade support 32a-d. The cutting edge 30a-d of a blade 28a-d is oriented forward in the direction of shaving S. The blade support 32a-d of a blade 28a-d is an elongated, bent piece of rigid material. In an example, the blade support 32a-d is a metal such as austenitic stainless steel.

Each cutting member 28a-d in the group of cutting members 28a-d is, in an example, resiliently mounted in a blade receiving section 31 of the razor cartridge 20. The blade receiving section 31 comprises a longitudinal space in the razor cartridge 20 that is sized to accommodate the group of cutting members 28a-d. At least one cutting member 28a of the group of cutting members 28a-d, up to all cutting members in the group of cutting members 28a-d may be resiliently mounted in the blade receiving section 31. In the illustrated example of **Fig. 2****,** the transverse inner sides of frame 21 comprise a plurality of holding slots 34a-d. Each holding slot 34a-d on the transverse inner sides is configured to receive and retain an end of one side of a blade support 32a-d of a cutting member 28a-d of the group of cutting members 28a-d so that the cutting members 28a-d of the group of cutting members 28a-d are held in the blade receiving section 31 with a substantially parallel inter-blade span in the transverse direction (-x to x). Therefore, as many holding slots 34a-d are provided in each transverse inner side of frame 21 as there are blades 33a-d.

Between the cutting member receiving section 31 and the handle (in a part adjacent to a handle connection, for example) there are, in examples, provided one or more transverse frame members 35 that are integrally formed with the frame 21. The transverse frame members 35 comprises a plurality of cutting member guides 36 provided as a plurality of protuberances aligned with the holding slots 34a-d on the transverse inner sides of the frame 21. The cutting member guides 36 function to regulate the parallel inter-blade span.

A cutting member guides 36 is provided on a portion of the transverse frame member 35 as a protrusion. For example, the cutting member guide 36 is provided as an injection-molded protrusion of the transverse frame member 35. For example, the cutting member guide 36 is integrally formed with the transverse frame member 35. In an example, each cutting member guide 36 of the plurality of cutting member guides 36 is aligned on a common axis of the at least one transverse frame member. In another example, each cutting member guide 36 of the plurality of cutting member guides 36 is aligned on a central axis of the at least one transverse frame member 35. In another example, at least one cutting member guide 36 is aligned away from a common axis or central axis 35 of the at least one transverse frame member 35.

A longitudinal skincare element 50 is held on an example longitudinal trailing assembly 49. In an example, the alternative razor cartridge comprises a trimming blade 53 which is held by a trimming blade support 54. A skilled person will appreciate that the example longitudinal trailing assembly 49 may be omitted without loss of generality. The cutting members 28a-d comprise blade supports 32a-d and their blades 33a-d are positioned in-between the cutting member guides 36.

In embodiments, the blade guides 36 are configured to support "bent blades" having a radiused portion in which the cutting edge is integral with (formed from the same piece of metal) as the blade support, as known to a skilled person. Blade guides 36 configured to support "bent blades" may, for example, comprise a curved upper portion configured to support or accommodate the radius portion of the "bent blade", for example.

**Figs. 4a-4c** are schematic views of a razor cartridge 20 according to other aspects. The depicted razor cartridges 20 comprise several magnetic elements 10 as described above. Although, in the embodiments of **Figs. 4a-4c****,** the razor cartridges 20 comprise two or three magnetic elements 10, it is to be understood that a razor cartridge 20 may only comprise one magnetic element 10 or more than three magnetic elements 10 according to any one of the described aspects. Additionally, it is to be understood combinations of aspects are possible, i.e. that embodiments are possible which, for instance, comprise a first magnetic element 10 according to any one of the magnetic elements 10 as illustrated in **Fig. 4a** (and/or **Fig. 4b** and/or **Fig. 1c**) and a second magnetic element 10 according to any one of the magnetic elements 10 as illustrated in **Fig. 4b** (and/or **Fig. 4a** and/or **Fig. 1c**).

According to an aspect depicted in **Fig. 4a****,** the razor cartridges 20 comprises three magnetic elements 10. A first magnetic element 10 is arranged at the leading longitudinal member 24. This is particularly advantageous as sufficient lubrication can be provided by initially manipulating the bulk properties (by the induced change) of that portion of the magnetic shaving aid which first comes in contact with the leading side of the razor cartridge 20. Furthermore, hairs can be pre-oriented and lifted-up before the first blade 28a (and subsequently to following blades 28b-d) start the cutting operation and removing the hair. A second magnetic element 10 is arranged at the trailing longitudinal member 25. This is particularly advantageous as used magnetic shaving aid including removed hair and debris can be collected behind the cutting members 28a-d and do not impair the ongoing shaving operation. Furthermore, the rinsabilty after the shaving operation can be improved by leading the magnetic shaving aid away from the cutting members 28a-d to the trailing side of the razor cartridge 20 which eases cleaning. This also prevents or at least reduces agglomeration and deposition of shaving aid, hairs and debris on or between the cutting members 28a-d which can improve the shaving efficiency.

In an aspect, the first magnetic element 10 is arranged at the first skin contacting element 12. Preferably, the first magnetic element 10 is attached to or integrated into the first skin contacting element 12. Alternatively, the first magnetic element 10 may be arranged between the first skin contacting element 12 and the frame 21. In an aspect, the second magnetic element 10 is arranged at the second skin contacting element 14. In particular, the second magnetic element 10 is attached to or integrated into the second skin contacting element 14. Alternatively, the second magnetic element 10 may be arranged between the second skin contacting element 14 and the frame 21. In **Fig. 1a****,** the first magnetic element 10 is thereby integrated into the first skin contacting element 12. That means it is arranged above a skin contacting surface of the first skin contacting element 12 in use. Thereby the first magnetic element 10 may not come into direct contact with the magnetic shaving aid and/or the skin surface K which prevents contamination of the first magnetic element 10. In **Fig. 1a****,** the second magnetic element 10 is arranged on a skin contacting surface of the second skin contacting element 14. For example, the second magnetic element 10 may form the skin contacting surface of the second skin contacting element 14 or at least a portion thereof. In another example, the second magnetic element 10 may protrude from the skin contacting surface of the second skin contacting element 14 towards the skin surface K to be closer to the skin surface K and/or the magnetic shaving aid as it is shown in **Fig. 4a****.** This may result in an enhanced strength of the induced change as the magnetic element 10 is closer to the skin surface K and/or the magnetic shaving aid. Furthermore, the induced change can be conducted locally more focused. Thereby used shaving aid including removed hair and debris can be collected easier at the trailing longitudinal member 25, i.e. behind the cutting members 28a-d. In other embodiments the respective other magnetic element 10 or both magnetic elements 10 may arranged above or at the respective skin contacting surface of a skin contacting element 12, 14. This may account for any magnetic elements 10 (i.e. also for a third, a fourth, a fifth, etc.).

A third magnetic element 10 is provided above the cutting members 28a-d (see, **Fig. 4a****).** Thereby, it is advantageous if the magnetic element 10 extends at least along a distance in shaving direction S in the area of cutting member receiving section 31 in order to reliably lift up hairs in the operation area of the cutting members 28a-d and to keep the magnetic shaving aid in a during-shave state during the cutting operation. For example, those magnetic elements 10 of **Figs. 4a** and **4c** which are arranged directly above the cutting members 28a-d between the leading longitudinal member 24 and the trailing longitudinal member 25 extend substantially along said advantageous distance. Also the magnetic element 10 of **Fig. 4b** which is arranged directly above the cutting members 28a-d between the leading longitudinal member 24 and the trailing longitudinal member 25 extends along said advantageous distance but extends further to towards the leading longitudinal member 24 which improves pre-lifting and pre-orienting of hairs. The term "directly above" describes a position which is above the related element in a direction orthogonal to the shaving direction S, whereas the term "directly adjacent" describes a position which is next to the related element without any further elements being arranged in between. In particular, the third magnetic element 10 may be arranged in or adjacent to the cutting member receiving section 31. In the example of Fig. 4a, the third magnetic element 10 is attached to an inner side of the frame 21 facing towards the skin surface K. More specifically, third magnetic element 10 is attached to an inner side of the platform member 22 facing towards the skin surface K. However, in other embodiments, it is also possible to arrange a magnetic element 10 above the cutting member receiving section 31, in particular above the inner side of the platform member 22 as it is shown in the example of **Figs. 4b-4c****.**

In another embodiment which is illustrated in **Fig. 4b****,** the razor cartridge 20 comprises two magnetic elements 10. A first magnetic element 10 is arranged, similarly as the third magnetic element 10 of the embodiment according to **Fig. 4a****,** above the cutting members 28a-d. However, in comparison to the third magnetic element 10 of **Fig. 4a****,** the first magnetic element 10 of **Fig. 4b** is arranged distanced from the inner side of the platform member 22 in an upper direction and extends further forward towards the guard member 23. Alternatively, the first magnetic element 10 may for instance, only extend in a forward region, i.e. before the cutting members 28a-d but at the same position in a direction orthogonal to the shaving plane S. A second magnetic element 10 is arranged, similarly as the second magnetic element 10 of the embodiment according to **Fig. 4a****,** at the trailing longitudinal member 25. However, in comparison to the second magnetic element 10 of **Fig. 4a****,** the second magnetic element 10 of **Fig. 4b** is rotated by 90 degrees and arranged at a side of the trailing longitudinal member 25 which faces in a direction opposite to the shaving direction S. In other words, the first magnetic element 10 can be described as having a first portion and a second portion. The first portion or forward portion of the first magnetic element 10 is arranged above the leading longitudinal member 24 in a direction orthogonal to the shaving plane S. The second portion or rearward portion of the first magnetic element 10 is arranged above cutting members 28a-d in a direction orthogonal to the shaving plane S. The first portion of the magnetic element 10 can "activate" the skin and the hair and prepare them for shaving, i.e. it improves pre-lifting and pre-orienting of hairs. The second portion of the first magnetic element 10 has a comparable effect as the third magnetic element 10 of **Fig. 4a****.** It will continue to provide the magnetic field, thus keeping the magnetic shaving aid in a during-shave state 31 and reliably lifting up hairs in the operation area of the cutting members 28a-d.

In another embodiment which is illustrated in **Fig. 4c****,** the razor cartridge 20 comprises two magnetic elements 10. A first magnetic element 10 is arranged, similarly as the first magnetic element 10 of the embodiment according to **Fig. 4b****,** above the cutting members 28a-d. However, in comparison to the first magnetic element 10 of **Fig. 4b****,** the first magnetic element 10 of **Fig. 4c** only extends along a distance in shaving direction S within the operation area of the cutting members 28a-d as explained above. A second magnetic element 10 is arranged, similarly as the second magnetic element 10 of the embodiment according to **Fig. 4a****,** at the trailing longitudinal member 25. However, in comparison to the second magnetic element 10 of **Fig. 4a****,** the second magnetic element 10 of **Fig. 4c** is arranged distanced from the second skin contacting element 14 in an upper direction away from the skin surface K. In particular, the second magnetic element 10 is arranged on an upper side of the razor cartridge 20 which faces away from the skin surface K.

In another aspect, the razor cartridge 20 comprises a comb 16 (see, **Figs. 1c** and **4a-****4c).** The comb 16 is arranged at the leading longitudinal member 24. In particular, the comb 16 is arranged at a forward portion of the leading longitudinal member 24, for instance, at the guard member 23 (see, **Fig. 1a**). The comb 16 may be arranged before a magnetic element 10, in particular before the first magnetic element 10 (i.e. the most forward magnetic element10 which is closest to the leading longitudinal side) and/or adjacent to a magnetic element 10. Additionally or alternatively, the magnetic element 10 may be arranged above the comb 16 (see, **Figs. 1b** and **1c**). The comb 16 is configured to guide and pre-orient hairs mechanically to be subsequently lifted up magnetically by the magnetic elements 10. This may additionally improve the shave performance.

The comb 16 may be an additional element which is attached to the frame 21, to the leading longitudinal member 24, to the first skin contacting element 12 and/or to the guard member 23. The attachment may be realized by an adhesive connection such as a glue connection, by a press-fit connection, by a clip connection or any other suitable connection. Alternatively, the comb 16 may be provided as a feature of the leading longitudinal member 24, of the guard member 23 or of the first skin contacting element 12. That means, the comb 16 may be integrally formed with one of these parts. Thereby, the comb 16 may be realized as protrusions in the guard member 23 or in the first skin contacting element 12. The comb 16 may be configured as a micro comb or nano comb.

In another aspect, the razor cartridge 20 comprises a one or more channels 18. A schematic channel 18 is illustrated in **Fig. 4b** with dashed lines. The channel 18 is arranged above the cutting members 28a-d and configured to guide used magnetic shaving aid including removed hairs and debris away from the cutting members 28a-d. Therefore, the one or more channels 18 are oriented from the leading longitudinal member 24 towards the trailing longitudinal member 25. This is particularly advantageous when a magnetic element 10 is arranged at trailing longitudinal member 25 and configured to draw the used shaving aid from the cutting member receiving section 31 the trailing longitudinal member 25. Thereby rinsability of the razor cartridge 20 can be improved and the risk of clogging can be reduced.

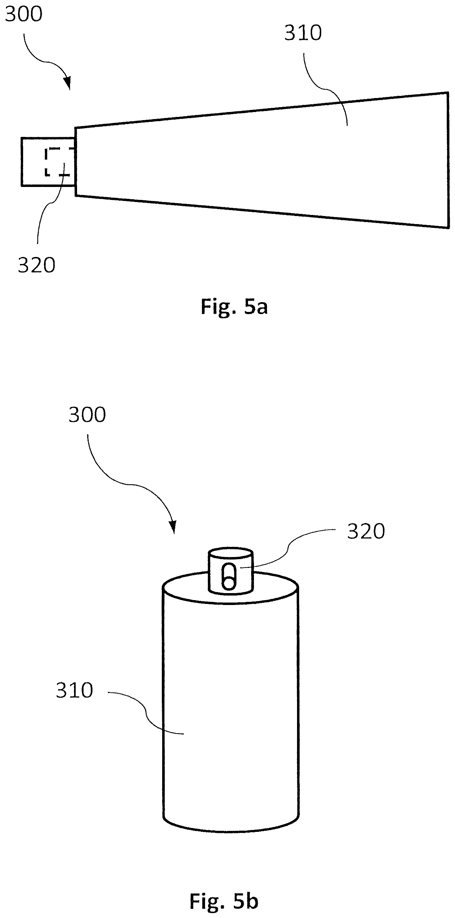

**Figs. 5a-5b** show two different embodiments of a hand-held dispenser 300 including the magnetic shaving aid composition. The hand-held dispenser 300 comprises a container 310 and a manually operable dispensing means 320. The container 310 contains a magnetic shaving aid composition comprises water and one or more surfactants comprising at least one magnetic surfactant. In some aspects, the magnetic shaving aid may be foamable. In general, the magnetic shaving aid is dispensed via the manually operable dispensing means 320. When operated by a user, the manually operable dispensing means 320 dispenses a portion of the magnetic shaving aid composition in response to positive pressure in the container 310. The positive pressure is either build up by the user when operating the manually operable dispensing means 320 or present in the container 310 due to the presence of a propellant within the magnetic shaving aid composition.

In an example, the magnetic shaving aid composition is configured as a gel. In an example, the magnetic shaving aid composition is configured as a cream. The gel may be foamable when being mixed with water. The cream may be foamable when being mixed with water. In general, the magnetic shaving aid composition includes a surfactant to contribute cleansing and thickening properties. Furthermore, a surfactant is present in the magnetic shaving aid composition to adjust the surface tension of the magnetic shaving aid composition, in particular the surfactant can lower the surface tension of the magnetic shaving aid composition and may cause it to foam as described above. The presence of the magnetic surfactant confers the ability of the magnetic shaving aid composition to conduct an induced change in response to magnetic effects provided by a magnet element 10 of a razor 100 as described above. The induced change can significantly alter the properties of the magnetic shaving aid composition. That means, the magnetic shaving aid composition can be physically moved and its bulk properties can be manipulated under the influence of magnetic fields.

**Fig. 5a** illustrates a first embodiment, wherein the hand-held dispenser 300 is a tube type dispenser. Thereby, the container 310 is designed as a tube. The manually operable dispensing means 320 is designed as a dosage opening. The tube is easily compressible when a user exerts external mechanical force. Thereby, positive pressure can be built up in the container 310 which leads to the effect that the magnetic shaving aid composition can be dispensed. In other words, the manually operable dispensing means 320 is operated by applying mechanical pressure externally on the tube container 310 in order to dispense the magnetic shaving aid composition.

**Fig. 5b** illustrates a second embodiment, wherein the hand-held dispenser 300 is a fingertip type dispenser. Thereby, the container 310 is designed as a can. The manually operable dispensing means 320 is designed as a pressure button. The manually operable dispensing means 320 is operated by pushing down the pressure button such that the magnetic shaving aid composition is forced out of the container 310 by fluidically coupling the inside of the container 310 with the environment (i.e. ambient air at atmospheric pressure) via the manually operable dispensing means 320. The positive pressure in the container is provided by a propellant in the magnetic shaving aid composition. The propellant can be initial ingredient of the magnet shaving aid composition or may be added from an extra propellant gas reservoir which is fluidically coupled to the inside of the container 310 when operating the manually operable dispensing means 320.

In an aspect (not shown), the hand-held dispenser 300 comprises structures which are adapted to the properties of the magnetic shaving aid composition such that the desirable properties of a pre-shave state of the magnetic shaving aid composition are achieved during or after the magnetic shaving aid composition is delivered to a skin surface K. For instance, the container 310 may comprise a coating or a layer which magnetically insulates the inside of the container 310 from the outside of the container 310.

In an aspect (not shown), the hand-held dispenser 300 comprises one or more magnetic elements 10 configured and arranged to apply a magnetic field to the magnetic shaving aid composition during or after dispensing the magnetic shaving aid composition. For instance, the magnetic elements 10 may be arranged at the manually operable dispensing means 320. Thereby, during dispensing the magnetic element 10 (when activated) can elicit an induced change in the magnetic shaving aid composition to a during-shave state which eases dispensing and distribution of the magnetic shaving aid composition on the skin surface K due to improved lubrication properties of the magnetic shaving aid composition in the during-shave state. Due to the fact that the induced change is reversible when the magnetic shaving aid composition is not any more under the influence of magnetic fields as described above, another induced change can be elicited in the magnetic shaving aid composition to a pre-shave state can be provided in order to achieve the desired properties before starting the shaving operation. This can be achieved by removing the hand-held dispenser 300 from the dispensed magnetic shaving aid composition or by simply deactivating the magnetic element(s) 10. However, during the time the magnetic fields where activated at least some hairs which were already in contact with the magnetic shaving aid composition may already have been be pre-oriented upwardly from the skin surface K before starting the shaving operation. Thereby, at least some hairs may remain lifted up to at least some extent before starting the shaving operation.

In aspects, the magnetic surfactant is an ionic compound or a salt. In preferred aspects, the ionic compound or salt is an ionic liquid. Ionic liquids are considered to be non-toxic to mammalian cells and skin and therefore enhance the comfort for a user.

In aspects, the magnetic surfactant comprises a positively charged nitrogen-containing compound and/or a negatively charged paramagnetic iron compound. In aspects, the magnetic surfactant is a ferrate-based ionic compound. In preferred aspects, the magnetic surfactant is a ferrate-based ionic liquid, i.e. an ionic liquid containing ferrates. An ionic liquid containing ferrates can also be denoted a magnetic ionic liquid (MIL). In other words, the magnetic surfactant can be a magnetic ionic liquid surfactant (MILS), preferably comprising a ferrate-based ionic liquid surfactant. In aspects, the magnetic surfactant is selected from the group consisting of 1-methyl-trimethylimidazolium tetrachloroferrate, dodecyltrimethylammonium bromotrichloroferrate, or mixtures thereof. Magnetic surfactants are affected on the molecular scale, where the magnetic field alters their surfactant properties, rather than just at the macro scale inducing movement. This can change surface tension, alter foaming behaviour etc. The key distinction therefore is the modulation of molecular surfactant properties.

In one aspect, the amount of the magnetic surfactant relative to the total weight of the magnetic shaving aid composition is at least 3 wt.-%, more specifically at least 5 wt.-%. An amount of at least 3 wt.-%, more specifically at least 5 wt.-% may ensure to achieve the desired effects explained above with regards to the induced change. In higher concentrations of magnetic surfactant, the response to magnetic forces is augmented. In other embodiments, the amount of magnetic surfactant may be between 3 wt.-% and 80 wt.-%, specifically between 5 wt.-% and 60 wt.-%, more specifically between 5 wt.-% and 20 wt.-% and in particular between 5 wt.-% and 10 wt.-%, relative to the total weight of the magnetic shaving aid composition.

In aspects, the magnetic shaving aid composition further comprises one or more of, a humectant, an emulsifying agent, an emulsion stabilizer, a neutralizer, a skin conditioning agent, a propellant gas, a preservative, an oil or fatty compound, a non-magnetic surfactant, an emollient, a pH-adjusting agent, an antioxidant, chelating agents, a fragrant, a rheology modifier or thickener, a gelling agent (capable of generating a gelling effect) and a colorant.

In embodiments, the magnetic shaving aid composition comprises at least a humectant, an emulsifying agent or an emulsifying system, one or more emulsion stabilizer, and one or more skin conditioning agents in addition to the water and the one or more surfactants comprising at least a magnetic surfactant. If the magnetic shaving aid composition comprises an emulsifying system comprising an emulsifier and a co-emulsifier. In preferred embodiments, two emulsion stabilizers may be used. In some aspects, the magnetic shaving aid composition further comprises a neutralizer. In some aspects, one or more neutralizers selected from the group consisting of sodium hydroxide, potassium hydroxide, and mixtures thereof may be used. In a preferred embodiment, hydroxide in a 50% solution and/or potassium hydroxide in a 50% solution may be used. In particular, the amount of neutralizer is of 0.1-10 wt.-%, relative to the total weight of the magnetic shaving aid composition The cosmetic composition may include a neutralizer to set the pH value of the magnetic shaving aid composition at levels suitable for skin care applications. The magnetic shaving aid composition has a pH value of 4.5 to 10.0, specifically 5.0 to 7.0 and most specifically of 5.5 or 6.0.

In embodiments, the magnetic shaving aid composition comprises, relative to the total weight of the magnetic shaving aid composition:
- the one or more surfactants comprising at least one magnetic surfactant in an amount of 3-80 wt.-%, specifically 5-60 wt.-%, more specifically between 5 wt.-% and 20 wt.-% and in particular 5-10 wt.-%, relative to the total weight of the magnetic shaving aid composition;
- a humectant in an amount of 1-15 wt.-%, specifically 1-10 wt.-%, relative to the total weight of the magnetic shaving aid composition;
- an emulsifying agent in an amount of 0.5-15 wt.-%, specifically 0.5-10 wt.-%, relative to the total weight of the magnetic shaving aid composition;
- preferably a neutralizer in an amount of 0.1-10 wt.-%, relative to the total weight of the magnetic shaving aid composition;
- a skin conditioning agent in an amount of 0.3-30 wt.-%, specifically 0.3-25 wt.-%, relative to the total weight of the magnetic shaving aid composition;
- water in particular in an amount of 10-90 wt.-%, specifically 20-80 wt.-%, relative to the total weight of the magnetic shaving aid composition.

In one embodiment, the magnetic shaving aid composition additionally comprises one or more preservatives. In one aspect, the magnetic shaving aid composition comprises more than one preservative, for instance a preservation system. In aspects, the magnetic shaving aid composition is dispensed in the form of a shaving cream. In aspects, the shaving cream is dispensed from a hand-held dispenser 300 comprising a tube container 310 as described above. In aspects, the magnetic shaving aid composition may comprise propellant gas such as isopentane or isobutane, but not necessarily.

In one embodiment, the magnetic shaving aid composition additionally comprises a propellant gas such as isopentane or isobutane in particular in an amount of 0.5-20 wt.-%, more specifically 1-10 wt.-%. In aspects, the magnetic shaving aid composition is dispensed in the form of a shaving gel. In aspects, the shaving gel is dispensed from a hand-held dispenser 300 comprising a can container 310 as described above. In aspects, the magnetic shaving aid composition may comprise one or more preservatives, but not necessarily.

Providing the magnetic shaving aid composition is in a compact structure-like form such as a liquid, a cream,a gel or a foam, in particular a cream, gel or foam. Compact structure-like form is preferred as it can sustain magnetic forces without being torn apart. In some aspects, it is possible to provide the magnetic shaving aid composition in form of a shaving foam. In embodiments, the magnetic shaving aid composition is a foamable magnetic shaving aid.

In embodiments, the magnetic shaving aid composition additionally comprises one or more non-magnetic surfactant in particular selected from the group consisting of anionic surfactants, cationic surfactants, amphoteric surfactants, nonionic surfactants, and mixtures thereof. Alternatively or additionally, the magnetic shaving aid composition additionally comprises a non-magnetic surfactant selected from the group consisting of fatty acids and/or their salts or amphoteric surfactants, anionic surfactants, nonionic surfactants, and mixtures thereof. In general, the addition of a non-magnetic surfactant contributes to cleansing, thickening, emulsifying, and foaming properties of the magnetic shaving aid composition. In particular, when present in the composition, the amount of non-magnetic surfactant(s) is of 0.5-60 wt.-%, specifically 0.5-40 wt.-%, relative to the total weight of the magnetic shaving aid composition.

In embodiments, the magnetic shaving aid composition comprises a fatty compound selected from the group consisting of fatty alcohols, fatty acids or esters, in particular comprising a fatty alcohol or a fatty acid, and mixtures thereof.

In some aspects, the fatty acids and/or their salts are selected from the group consisting of palmitic acid, myristic acid, stearic acid, lauric acid, coconut fatty acids, sodium stearate, and mixtures thereof.

In some aspects, the amphoteric surfactants are selected from the group consisting of cocamidopropyl betaine, coco-betaine, and mixtures thereof.

In some aspects, the anionic surfactants are selected from the group consisting of sodium laureth sulphate, sodium methyl cocoyl taurate, sodium cocoyl isethionate, and mixtures thereof. In some aspects, the nonionic surfactants are selected from the group consisting of decyl glucoside, coco glucoside, and mixtures thereof.

In embodiments, the magnetic shaving aid composition comprises a humectant, in particular selected from the group consisting of glycereth-26, sorbitol, glycerin, and mixtures thereof. The magnetic shaving aid composition includes a humectant that act as hydroscopic moisturizer and attract water to provide hydrating properties that increase and maintain moisture in the skin and/or hair. The humectant may also contribute to lubrication.In particular, the amount of humectant(s) of 1-15 wt.-%, specifically 1-10 wt.-%, relative to the total weight of the magnetic shaving aid composition.

In embodiments, the magnetic shaving aid composition comprises an emulsifying agent and/or an emulsifying system, in particular selected from the group consisting of oleth-20, glyceryl stearate, and mixtures thereof. The magnetic shaving aid composition includes an emulsifying agent to provide stabilization of emulsion and homogeneity of the blend. The emulsifying agent forms and stabilizes an emulsion by keeping hydrophilic and hydrophobic materials together in a homogeneous mixture. In particular embodiments, an emulsifier and a co-emulsifier may be used. In particular, the amount of emulsifying agent(s) may of 0.5-15 wt.-%, specifically 0.5-10 wt.-%, relative to the total weight of the magnetic shaving aid composition.

In embodiments, the magnetic shaving aid composition comprises an emulsion stabilizer, in particular selected from the group consisting of cetyl alcohol, behenyl and cetearyl alcohols or nonionic polymers such as hydroxypropylcellulose, hydroxyethylcellulose, hydroxypropyl methylcellulose, and mixtures thereof.

In embodiments, the magnetic shaving aid composition comprises a skin conditioning agent, in particular selected from the group consisting of oils, vegetable butters, plant extracts, skin care/healing substances, and mixtures thereof. The cosmetic composition includes a skin conditioning agent to contribute skin care benefits. In particular embodiments, two skin conditioning agents may be used. In aspects, the oils are selected from the group consisting of vegetable oils such as sunflower seed oil, or synthetic oils such as octyldodecanol, caprylic/capric triglyceride, paraffinum liquidum, and mixtures thereof. In aspects, the vegetable butters are selected from the group consisting of shea butter, cocoa butter, and mixtures thereof. In aspects, the plant extracts are selected from the group consisting of camellia sinensis extract, salvia officinalis extract, and mixtures thereof. To those skilled art, a large number of plant extracts is known to choose from, any of which could alternatively or additionally be selected based on their properties, for instance antioxidant, anti-inflammatory, soothing, hydrating, moisturizing, refreshing and/or cooling properties. In aspects, the skin care/healing substances are selected from the group consisting of panthenol, polyquaterniums, and mixture thereof. In particular, the skin conditioning agent(s) is/are present in an amount of 0.3-30 wt.-%, specifically 0.3-25 wt.-%.

In some embodiments, the magnetic shaving aid composition comprises one or more pH adjusters such as citric acid, one or more chelating agents such as trisodium ethylenediamine disuccinate or phytic acid, one or more antioxidants such as tocopheryl acetate and/or one or more fragrances, and mixtures thereof. In some embodiments, the magnetic shaving aid composition comprises one or more emollients such as PEG-14M and/or one or more colorants.
These ingredients are ingredients which either contribute in the build up of the magnetic shaving aid composition (e.g. to achieve a compact, homogenized product) or for protection or to improve the aesthetics of the magnetic shaving aid composition. The quantities vary depending on the desired effect of the final formulation (i.e. the magnetic shaving aid composition). It is common to be used in less than 1 wt.-%, specifically less than 0.7 wt.-% or at amounts that allow their activity to be exhibited while being compliant with safety protocols for skin care/cosmetic applications.

### Particular embodiment 1:

According to a first particular embodiment, the magnetic shaving aid composition of the present invention is in the form of a shaving gel and comprises:

| **Shaving gel** | | | |
|---|---|---|---|
| **Raw material familly** | **Example** | **wt.-%** | **Function** |
| Emulsifiers | Oleth-20 | 1-10 | Emulsifier |
| Propellant gas | Isopentane & Isobutane | 1-10 | Propellant |
| Nonionic polymer | Hydroxypropylcellulose, Hydroxyethylcellulose | 0,1-5 | Emulsion stabiliser |
| Combo of fatty acids | Palmitic acid, Myristic acid, Stearic acid | 2-15 | Surfactant, cleansing properties |
| Water soluble resin | PEG-14M | 0,1-1 | Emollient, lubricant |
| Neutralizers | Sodium Hydroxide 50% solution | 0,1-10 | Neutralizer |
| Humectants | Glycereth-26, Glycerine | 1-10 | Humectant, Lubricant |
| Vegetable oils & synthetic oils | Vegetable oils: Sunflower seed oil Synthetic oils: Octyldodecanol, Caprylic/Capric Triglyceride, Paraffinum Liquidum | 0,1-10 | Skin & hair conditioning |
| pH adjuster | Citric acid | 0,1-5 | pH adjuster if needed |
| Antioxidants | Tocopheryl Acetate | 0,1-3 | Antioxidant, skin conditioner |
| Chelating agent | Trisodium Ethylenediamine Disuccinate, Phytic acid | 0,1-1 | Chelating agent |
| Fragrance | Perfume | 0,1-3 | Perfuming |
| Magnetic surfactant | 1-methyl-trimethylimidazolium tetrachloroferrate, dodecyltrimethylammonium bromotrichloroferrate | 5-10 | Surfactant |
| Vegetable butters | Shea butter, Cocoa butter | 0,1-5 | Skin & hair conditioning |
| Plant Extracts | Camellia Sinensis extract, Salvia Officinalis extract | 0,1-5 | |
| Skin and hair conditioning agents | Panthenol, Polyquaterniums etc | 0,1-10 | Skin & hair conditioning |
| Color | Coloring agent(s) | 0,0001-0,01 | Coloring agent |
| Water | Aqua | add to 100% | Solvent |

### Particular embodiment 2:

According to a second particular embodiment, the magnetic shaving aid composition of the present invention is in the form of a shaving cream and comprises:

| **Shaving cream** | | | |
|---|---|---|---|
| **Raw material familly** | **Example** | **wt.-%** | **Function** |
| Combo of fatty acids and/or their salts | Fatty acids: Lauric acid, Palmitic acid, Myristic acid, Stearic acid Coconut fatty acids Salts: Sodium Stearate | 10-60 | Surfactant, cleansing properties |
| Neutralizers | Potassium Hydroxide 90% flakes, Sodium Hydroxide 50% solution | 0,1-10 | Neutralizer |
| Emulsifiers/C o-emulsifiers | Glyceryl Stearate | 0,5-5 | Co-emulsifier/Structurant |
| Saturated fatty alcohols | Cetyl Alcohol | 0,5-5 | Structurant |
| Nonionic polymer | Hydroxyethylcellulose, Hydroxypropyl Methylcellulose | 1-10 | Emulsion stabiliser |
| Amphoteric surfactants | Cocamidopropyl Betaine, Coco-betaine | 1-10 | Surfactant, cleansing properties |
| Magnetic surfactant | 1-methyl-trimethylimidazolium tetrachloroferrate, dodecyltrimethylammonium bromotrichloroferrate | 5-10 | Surfactant |
| Humectants | Glycerine | 1-15 | Humectant, skin and hair conditioner |
| Antioxidants | Tocopheryl Acetate | 0,1-3 | Antioxidant, skin conditioner |
| Preservative | Preservative | 0,1-5 | Preservative |
| Chelating agent | Trisodium Ethylenediamine Disuccinate, Phytic acid | 0,1-1 | Chelating agent |
| Fragrance | Perfume | 0,1-3 | Perfuming |
| Vegetable oils & synthetic oils | Vegetable oils: Sunflower seed oil Synthetic oils: Octyldodecanol, Caprylic/Capric Triglyceride | 0,1-10 | Skin & hair conditioning |
| Vegetable butters | Shea butter, Cocoa butter | 0,1-5 | Skin & hair conditioning |
| Plant Extracts | Camellia Sinensis extract, Salvia Officinalis extract | 0,1-5 | |
| Skin and hair conditioning agents | Panthenol, Polyquaterniums etc | 0,1-10 | Skin & hair conditioning |
| pH adjuster | Citric acid | 0,1-5 | pH adjuster if needed |
| Water | Aqua | add to 100% | Solvent |

According to another aspect, a razor kit is disclosed (not shown). The razor kit comprises a razor 100 and a hand-held dispenser 300 as described above.

According to another aspect, a method is disclosed for avoiding skin irritation and improving the shave efficiency during a shaving operation by a user with a razor 100 in combination with a magnetic shaving aid. The method includes providing a razor 100 having a razor cartridge 20 and at least one magnetic element 10. Then providing a magnetic shaving aid consisting of a magnetic shaving aid composition comprising a magnetic surfactant in a pre-shave state. Then applying the magnetic shaving aid to a skin surface K. Then performing a shaving operation with the razor 100 on the skin surface K. Then inducing a change in the magnetic shaving aid by applying a magnetic field in the magnetic shaving aid to bring the magnetic shaving aid from the pre-shave state to a during-shave state during performing the shaving operation.

In some aspects, the magnetic field interacts with the magnetic surfactant to bring the magnetic shaving aid in the during-shave state.

In some aspects, the magnetic field is applied to the magnetic shaving aid by drawing the razor 100 over the shaving surface with the magnetic elements 10 in proximity to the magnetic shaving aid. Thereby, the magnetic element 10 provides a static magnetic field.

In some aspects, the magnetic element 10 is an electromagnet and the magnetic field is activated by turning on a switch 260 of the razor 100 during performing the shaving operation. In an aspect, the razor 100 comprises a razor handle 200. In some aspects, the switch 260 is arranged at the razor handle 200.

In some aspects, performing the shaving operation involves lifting up and/or pre-orienting hairs on the skin surface K with a comb 16 arranged at a leading longitudinal member 24 of the razor cartridge 20 before or during applying a magnetic field in the magnetic shaving.

In some aspects, the induced change comprises exerting a magnetic pulling force on the magnetic surfactant to effect a physical movement of the magnetic shaving aid.

In some aspects, the induced change comprises a micro-scale interaction of the magnetic fields with the magnetic surfactant altering the structuring properties of the magnetic surfactant to effect a reduction of surface tension and a reduction of viscosity of the magnetic shaving aid.

In some aspects, the magnetic shaving aid is applied to the skin surface K in the pre-shave state by operating a manually operable dispensing means 320 of a hand-held dispenser 300. The magnetic shaving aid may be provided in a container 310 of the hand-held dispenser 300.

In some aspects, operating the manually operable dispensing means 320 involves eliciting one or more induced changes between the during-shave state and the pre-shave state in the magnetic shaving aid to provide the magnetic shaving aid in the pre-shave state.

**Reference sign list**

| | | | |
|---|---|---|---|
| H | razor handle direction | 31 | cutting member receiving section |
| S | shaving direction | 32a-d | blade support |
| SP | shaving plane | 33a-d | blade |
| L | longitudinal axis/direction | 34a-d | holding slot |
| K | skin or skin surface | 35 | transverse frame member |
| 10 | Magnetic element | 36 | cutting member guide |
| 12 | first skin contacting element | 38a-d | resilient finger |
| 14 | second skin contacting element | 49 | longitudinal trailing assembly |
| 16 | comb | 50 | skin care element |
| 18 | channels | 53 | trimming blade assembly |
| 20 | razor cartridge | 54 | trimming blade support |
| 21 | frame | 100 | razor |
| 21a | first side portion | 200 | razor handle |
| 21b | second side portion | 210 | proximal portion |
| 22 | platform member | 220 | distal portion |
| 23 | guard member | 230 | coupling |
| 24 | leading longitudinal member | 240a,b | releasing mechanism |
| 25 | trailing longitudinal member | 250 | handle grip |
| 26 | first retainer | 260 | switch |
| 27 | second retainer | 300 | hand-held dispenser |
| 28a-d | cutting member | 310 | container |
| 29 | cap member | 320 | manually operable dispensing |
| 30a-d | cutting edge | | means |

It should be understood that the present invention can also (alternatively) be defined in accordance with the following configurations:
1. A razor cartridge (20) comprising:
   a frame (21) having a leading longitudinal member (24) and a trailing longitudinal member (25);
   one or more cutting members (28a-d) arranged between the leading longitudinal member (24) and the trailing longitudinal member (25);
   wherein
   at least one magnetic element (10) being configured and arranged to apply a magnetic field in a magnetic shaving aid comprising a magnetic surfactant and being applied to a skin surface (K) so as to elicit an induced change in the magnetic shaving aid between a pre-shave state and a during-shave state.
2. The razor cartridge (20) of configuration 1, wherein the induced change elicited by the magnetic element (10) involves physical movement of the magnetic surfactant and/or manipulation of bulk properties of the magnetic shaving aid.
3. The razor cartridge (20) of any one of the previous configurations, wherein the magnetic element (10) is configured to provide magnetic field strengths of 0.01-1 Tesla at a distance of 0.0cm to 2.0cm, specifically 0.0cm to 1.5cm, and most specifically 0.0cm to 1.0 cm from a surface of the magnetic element.
4. The razor cartridge (20) of any one of the previous configurations, wherein the magnetic element (10) is configured to provide a static magnetic field or an actively controlled magnetic field.
5. The razor cartridge (20) of any one of the previous configurations, wherein the induced change is reversible.
6. The razor cartridge (20) of any one of the previous configurations, wherein the induced change is elicited during a shaving operation, when the magnetic element (10) is brought in proximity to the magnetic shaving aid.
7. The razor cartridge (20) of any one of the previous configurations, wherein the magnetic element (10) is configured to keep the magnetic shaving aid in the during-shave state by constantly applying the magnetic field to the magnetic surfactant.
8. The razor cartridge (20) of configuration 7, wherein the magnetic element (10) is configured to reduce the surface tension of the magnetic shaving aid and to induce magnetic pulling forces in the magnetic shaving aid by applying the magnetic field to the magnetic surfactant.
9. The razor cartridge (20) of any one of the previous configurations, further comprising one or more skin contacting elements (12, 14) arranged at the leading longitudinal member (24) and/or at the trailing longitudinal member (25), in particular a first skin contacting element (12) and a second skin contacting element (14).
10. The razor cartridge (20) of configuration 9, wherein the magnetic element (10) is arranged at the skin contacting element (12, 14).
11. The razor cartridge (20) of any one of the previous configurations, wherein the magnetic element (10) is arranged at the leading longitudinal member (24) and/or at the trailing longitudinal member (25).
12. The razor cartridge (20) of any one of the previous configurations, further comprising a comb (16) which is arranged at the leading longitudinal member (24), in particular before the leading longitudinal member (24).
13. The razor cartridge (20) of configuration 12, wherein the magnetic element (10) is arranged at the comb (16), in particular wherein the magnetic element (10) is arranged above the comb (16).
14. The razor cartridge (20) of any one of the previous configurations, wherein the magnetic element (10) is arranged above the cutting members (28a-d) and optionally, above the leading longitudinal member (24), in particular between two cutting members (28a-d).
15. The razor cartridge (20) of any one of the previous configurations, wherein the frame (21) comprises one or more channels (18) above the cutting members (28a-d) and wherein the one or more channels (18) are oriented from the leading longitudinal member (24) to the trailing longitudinal member (25).
16. A razor (100) comprising:
   a razor handle (200); and
   a razor cartridge (20), wherein the razor cartridge (20) is coupled to the razor handle (200);
   wherein
   at least one magnetic element (10) being configured and arranged to apply a magnetic field in a magnetic shaving aid comprising a magnetic surfactant and being applied to a skin surface (K) so as to elicit an induced change in the magnetic shaving aid between a pre-shave state and a during-shave state.
17. The razor (100) of configuration 16, wherein the magnetic element (10) is attached to the razor handle (200), in particular wherein the magnetic element (10) is arranged at a distal portion (220) of the razor handle (200).
18. The razor (100) of configuration any one of configurations 16 or 17, wherein the razor cartridge (20) is either releasably attached to the razor handle (200) via a pivotable or non-pivotable coupling (230), integrally formed with the razor handle (200) via a non-pivotable coupling (230), or integrally formed with the razor handle (200) via a pivotable coupling (230).
19. The razor (100) of any one of configurations 16 to 18, wherein razor cartridge (20) is any one of the configurations 1 to 15.
20. The razor (100) of any one of configurations 16 to 19, wherein the razor handle (200) comprises a switch (260) which is configured and arranged to control the magnetic element (10).
21. A hand-held dispenser (300) comprising a container (310) containing a magnetic shaving aid composition, in particular a foamable magnetic shaving aid composition, the hand-held dispenser (300) further comprising a manually operable dispensing means (320) which, when operated, dispenses a portion of the magnetic shaving aid composition in response to positive pressure in the container (310), wherein said positive pressure is a) build up by the user when operating the manually operable dispensing means (320) or b) present in the container (310) due to the presence of a propellant within the magnetic shaving aid composition;
   wherein the magnetic shaving aid composition comprises water and one or more surfactants comprising at least one magnetic surfactant.
22. The hand-held dispenser (300) of configuration 21, wherein the hand-held dispenser (300) is a tube type dispenser, wherein the container (310) is designed as a tube and wherein the manually operable dispensing means (320) is a dosage opening.
23. The hand-held dispenser (300) of configuration 21, wherein the hand-held dispenser (300) is a fingertip type dispenser, wherein the container (310) is designed as a can and wherein the manually operable dispensing means (320) is a pressure button.
24. The hand-held dispenser (300) of any of configurations 21 to 23, further comprising structures which are adapted to the properties of the magnetic shaving aid composition such that the desirable properties of a pre-shave state of the magnetic shaving aid composition are achieved during or after the magnetic shaving aid composition is delivered to a skin surface (K).
25. The hand-held dispenser (300) of any one of configurations 21 to 24, wherein the manually operable dispensing means (320) comprises one or more magnetic elements (10) configured and arranged to apply a magnetic field to the magnetic shaving aid composition during or after dispensing the magnetic shaving aid composition, such that the desired properties of the magnetic shaving aid composition in the pre-shave state and/or the during-shave state are achieved.
26. The hand-held dispenser (300) of any of configurations 21 to 25, wherein the magnetic surfactant is an ionic compound or a salt, in particular, wherein the ionic compound or salt is an ionic liquid.
27. The hand-held dispenser (300) of any one of configurations 21 to 26, wherein the magnetic surfactant comprises a positively charged nitrogen-containing compound and/or a negatively charged paramagnetic iron compound.
28. The hand-held dispenser (300) of any one of configurations 21 to 27, wherein the magnetic surfactant is a ferrate-based ionic compound, in particular, wherein the magnetic surfactant is a ferrate-based ionic liquid.
29. The hand-held dispenser (300) of any one of configurations 21 to 28, wherein the magnetic surfactant is selected from the group consisting of 1-methyl-trimethylimidazolium tetrachloroferrate, dodecyltrimethylammonium bromotrichloroferrate, or mixtures thereof.
30. The hand-held dispenser (300) of any one of configurations 21 to 29, wherein the amount of magnetic surfactant is between 3 wt.-% and 60 wt.-%, specifically between 4 wt.-% and 40 wt.-%, and in particular between 5 and 10 wt.-%, relative to the total weight of the magnetic shaving aid composition.
31. The hand-held dispenser (300) of any one of configurations 21 to 30, wherein the magnetic shaving aid composition comprises at least:
   - a humectant,
   - an emulsifying agent or an emulsifying system,
   - one or more emulsion stabilizer, and
   - one or more skin conditioning agents.
32. The hand-held dispenser (300) of any one of configurations 21 to 31, wherein the magnetic shaving aid composition further comprises one or more of:
   - a humectant,
   - an emulsifying agent,
   - an emulsion stabilizer,
   - a neutralizer,
   - a skin conditioning agent,
   - a propellant gas,
   - a preservative,
   - an oil or fatty compound,
   - a non-magnetic surfactant,
   - an emollient,
   - a pH-adjusting agent,
   - an antioxidant,
   - chelating agents,
   - a fragrant,
   - a rheology modifier
   - a gelling agent and
   - a colorant.
33. The hand-held dispenser (300) of configuration 32, wherein the magnetic shaving aid composition further comprises a neutralizer, in particular one or more neutralizers selected from the group consisting of sodium hydroxide, potassium hydroxide, and mixtures thereof.
34. The hand-held dispenser (300) of any one of configurations 21 to 33, wherein the magnetic shaving aid composition comprises, relative to the total weight of the magnetic shaving aid composition:
   - the one or more surfactants comprising at least one magnetic surfactant in an amount of 3-80 wt.-%, specifically 5-60 wt.-%, more specifically 5-20 wt.-% and in particular 5-10 wt.-% relative to the total weight of the magnetic shaving aid composition;
   - a humectant in an amount of 1-15 wt.-%, specifically 1-10 wt.-% relative to the total weight of the magnetic shaving aid composition;
   - an emulsifying agent in an amount of 0.5-15 wt.-%, specifically 0.5-10 wt.-% relative to the total weight of the magnetic shaving aid composition;
   - preferably a neutralizer in an amount of 0.1-10 wt.-% relative to the total weight of the magnetic shaving aid composition;
   - a skin conditioning agent in an amount of 0.3-30 wt.-%, specifically 0.3-25 wt.-% relative to the total weight of the magnetic shaving aid composition;
   - water in particular in an amount of 10-90 wt.-%, specifically 20-80 wt.-%, relative to the total weight of the magnetic shaving aid composition.
35. The hand-held dispenser (300) of any one of configurations 32 to 34, wherein the magnetic shaving aid composition is dispensed in the form of a shaving cream and wherein the magnetic shaving aid composition comprises one or more preservatives.
36. The hand-held dispenser (300) of any one of configurations 32 to 34, wherein the magnetic shaving aid composition is dispensed in the form of a shaving gel and wherein the magnetic shaving aid composition comprises a propellant gas such as isopentane or isobutane in particular in an amount of 0.5-20 wt.-%, more specifically 1-10 wt.-%.
37. The hand-held dispenser (300) of any one of configurations 21 to 34, wherein the magnetic shaving aid composition is dispensed in the form of a shaving cream, a shaving gel or a shaving foam, specifically in the form of a shaving gel or a shaving cream, in particular the magnetic shaving aid composition is foamable.
38. The hand-held dispenser (300) of any one of configurations 21 to 37, wherein the magnetic shaving aid composition comprises a non-magnetic surfactant selected from the group consisting of anionic surfactants, cationic surfactants, amphoteric surfactants, nonionic surfactants, and mixtures thereof, in particular the amount of non-magnetic surfactant(s) is of 0.5-60 wt.-%, specifically 0.5-40 wt.-%, relative to the total weight of the magnetic shaving aid composition.
39. The hand-held dispenser (300) of any one of configurations 21 to 37, wherein the magnetic shaving aid composition comprises a non-magnetic surfactant selected from the group consisting of fatty acids and/or their salts or amphoteric surfactants, anionic surfactants, nonionic surfactants, and mixtures thereof, in particular the amount of non-magnetic surfactant(s) is of 0.5-60 wt.-%, specifically 0.5-40 wt.-%, relative to the total weight of the magnetic shaving aid composition.
40. The hand-held dispenser (300) of any one of configurations 21 to 39, wherein the magnetic shaving aid composition comprises a fatty compound selected from the group consisting of fatty alcohols, fatty acids or esters comprising a fatty alcohol or a fatty acid, and mixtures thereof.
41. The hand-held dispenser (300) of any one of configurations 39 or 40, wherein the fatty acids and/or their salts are selected from the group consisting of palmitic acid, myristic acid, stearic acid, lauric acid, coconut fatty acids, sodium stearate, and mixtures thereof.
42. The hand-held dispenser (300) of any one of configurations 38 to 41, wherein the amphoteric surfactants are selected from the group consisting of cocamidopropyl betaine, coco-betaine, and mixtures thereof.
43. The hand-held dispenser (300) of any one of configurations 38 to 42, wherein the anionic surfactants are selected from the group consisting of sodium laureth sulphate, sodium methyl cocoyl taurate, sodium cocoyl isethionate, and mixtures thereof.
44. The hand-held dispenser (300) of any one of configurations 38 to 43, wherein the nonionic surfactants are selected from the group consisting of decyl glucoside, coco glucoside, and mixtures thereof.
45. The hand-held dispenser (300) of any one of configurations 21 to 44, wherein the magnetic shaving aid composition comprises a humectant selected from the group consisting of glycereth-26, sorbitol, glycerin, and mixtures thereof.
46. The hand-held dispenser (300) of any one of configurations 21 to 45, wherein the magnetic shaving aid composition comprises an emulsifying agent and/or an emulsifying system selected from the group consisting of oleth-20, glyceryl stearate, and mixtures thereof.
47. The hand-held dispenser (300) of any one of configurations 21 to 46, wherein the magnetic shaving aid composition comprises an emulsion stabilizer selected from the group consisting of cetyl alcohol, behenyl and cetearyl alcohols or nonionic polymers such as hydroxypropylcellulose, hydroxyethylcellulose, hydroxypropyl methylcellulose, and mixtures thereof.
48. The hand-held dispenser (300) of any one of configurations 21 to 46, wherein the magnetic shaving aid composition comprises a skin conditioning agent selected from the group consisting of oils, vegetable butters, plant extracts, skin care/healing substances, and mixtures thereof.
49. The hand-held dispenser (300) of configuration 48, wherein the oils are selected from the group consisting of vegetable oils such as sunflower seed oil, or synthetic oils such as octyldodecanol, caprylic/capric triglyceride, paraffinum liquidum, and mixtures thereof.
50. The hand-held dispenser (300) of any one of configurations 48 or 49, wherein the vegetable butters are selected from the group consisting of shea butter, cocoa butter, and mixtures thereof.
51. The hand-held dispenser (300) of any one of configurations 48 to 50, wherein the plant extracts are selected from the group consisting of camellia sinensis extract, salvia officinalis extract, and mixtures thereof.
52. The hand-held dispenser (300) of any one of configurations 48 to 51, wherein the skin care/healing substances are selected from the group consisting of panthenol, polyquaterniums, and mixture thereof.
53. The hand-held dispenser (300) of any one of configurations 21 to 52, wherein the magnetic shaving aid composition further comprises one or more pH adjusters such as citric acid, one or more chelating agents such as trisodium ethylenediamine disuccinate or phytic acid, one or more antioxidants such as tocopheryl acetate and one or more fragrances, and optionally, wherein the magnetic shaving aid composition further comprises one or more emollients such as PEG-14M and/or one or more colorants.
54. The hand-held dispenser (300) of any one of configurations 21 to 53, wherein the magnetic shaving aid composition has a pH value of 4.5 to 10.0, specifically 5.0 to 7.0 and most specifically of 5.5 or 6.0.
55. A razor kit comprising:
   a razor (100) of any one of configuration 16 to 20; and
   a hand-held dispenser (300) of any of configurations 21 to 54.
56. A method for avoiding skin irritation and improving the shave efficiency during a shaving operation by a user with a razor (100) in combination with a magnetic shaving aid comprising the steps of:
   a) providing a razor (100) having a razor cartridge (20) and at least one magnetic element (10);
   b) providing a magnetic shaving aid consisting of a magnetic shaving aid composition comprising a magnetic surfactant in a pre-shave state;
   c) applying the magnetic shaving aid to a skin surface (K);
   d) performing a shaving operation with the razor (100) on the skin surface (K);
   e) inducing a change in the magnetic shaving aid by applying a magnetic field in the magnetic shaving aid to bring the magnetic shaving aid from the pre-shave state to a during-shave state during performing the shaving operation.
57. The method of configuration 56, wherein the magnetic field interacts with the magnetic surfactant to bring the magnetic shaving aid in the during-shave state.
58. The method of any one of configurations 56 or 57, wherein the magnetic field is applied to the magnetic shaving aid by drawing the razor (100) over the shaving surface with the magnetic elements (10) in proximity to the magnetic shaving aid.
59. The method of any one of configurations 56 to 58, wherein the magnetic element (10) is an electromagnet and wherein the magnetic field is activated by turning on a switch (260) of the razor (100) during performing the shaving operation.
60. The method of any one of configurations 56 to 59, wherein performing the shaving operation involves lifting up and/or pre-orienting hairs on the skin surface (K) with a comb (16) arranged at a leading longitudinal member (24) of the razor cartridge (20) before or during applying a magnetic field in the magnetic shaving aid.
61. The method of any one of configurations 56 to 60, wherein the induced change comprises exerting a magnetic pulling force on the magnetic surfactant to effect a physical movement of the magnetic shaving aid.
62. The method of any one of configurations 56 to 61, wherein the induced change comprises a micro-scale interaction of the magnetic fields with the magnetic surfactant altering the structuring properties of the magnetic surfactant to effect a reduction of surface tension and a reduction of viscosity of the magnetic shaving aid.
63. The method of any one of configurations 56 to 62, wherein the magnetic shaving aid is applied to the skin surface (K) in the pre-shave state by operating a manually operable dispensing means (320) of a hand-held dispenser (300).
64. The method of configuration 63, wherein operating the manually operable dispensing means (320) involves eliciting one or more induced changes between the during-shave state and the pre-shave state in the magnetic shaving aid to provide the magnetic shaving aid in the pre-shave state.

## Claims

1. A razor cartridge (20) comprising:
a frame (21) having a leading longitudinal member (24) and a trailing longitudinal member (25);
one or more cutting members (28a-d) arranged between the leading longitudinal member (24) and the trailing longitudinal member (25);
wherein
at least one magnetic element (10) being configured and arranged to apply a magnetic field in a magnetic shaving aid comprising a magnetic surfactant and being applied to a skin surface (K) so as to elicit an induced change in the magnetic shaving aid between a pre-shave state and a during-shave state.

2. The razor cartridge (20) of claim 1, wherein the induced change elicited by the magnetic element (10) involves physical movement of the magnetic surfactant and/or manipulation of bulk properties of the magnetic shaving aid.

3. The razor cartridge (20) of any one of the previous claims, wherein the magnetic element (10) is configured to provide a static magnetic field or an actively controlled magnetic field.

4. The razor cartridge (20) of any one of the previous claims, further comprising one or more skin contacting elements (12, 14) arranged at the leading longitudinal member (24) and/or at the trailing longitudinal member (25), in particular a first skin contacting element (12) and a second skin contacting element (14), and, wherein the magnetic element (10) is arranged at the skin contacting element (12, 14), and/or wherein the magnetic element (10) is arranged above the cutting members (28a-d) and the leading longitudinal member (24), in particular between two cutting members (28a-d).

5. A razor (100) comprising:
a razor handle (200); and
a razor cartridge (20), wherein the razor cartridge (20) is coupled to the razor handle (200);
wherein
at least one magnetic element (10) being configured and arranged to apply a magnetic field in a magnetic shaving aid comprising a magnetic surfactant and being applied to a skin surface (K) so as to elicit an induced change in the magnetic shaving aid between a pre-shave state and a during-shave state.

6. The razor (100) of claim 5, wherein the magnetic element (10) is attached to the razor handle (200), in particular wherein the magnetic element (10) is arranged at a distal portion (220) of the razor handle (200).

7. The razor (100) of any one of claims 5 or 6, wherein razor cartridge (20) is any one of the claims 1 to 4.

8. A hand-held dispenser (300) comprising a container (310) containing a magnetic shaving aid composition, in particular a foamable magnetic shaving aid composition, the hand-held dispenser (300) further comprising a manually operable dispensing means (320) which, when operated, dispenses a portion of the magnetic shaving aid composition in response to positive pressure in the container (310), wherein said positive pressure is a) build up by the user when operating the manually operable dispensing means (320) or b) present in the container (310) due to the presence of a propellant within the magnetic shaving aid composition;
wherein the magnetic shaving aid composition comprises water and one or more surfactants comprising at least one magnetic surfactant, in particular in an amount of 3-80 wt.-%, specifically 5-60 wt.-%, more specifically 5-20 wt.-% and in particular 5-10 wt.-% relative to the total weight of the magnetic shaving aid composition.

9. The hand-held dispenser (300) of claim 8, wherein the manually operable dispensing means (320) comprises one or more magnetic elements (10) configured and arranged to apply a magnetic field to the magnetic shaving aid composition during or after dispensing the magnetic shaving aid composition, such that the desired properties of the magnetic shaving aid composition in the pre-shave state and/or the during-shave state are achieved.

10. The hand-held dispenser (300) of any of claims 8 or 9, wherein the magnetic surfactant is an ionic compound or a salt, in particular, wherein the ionic compound or salt is an ionic liquid.

11. The hand-held dispenser (300) of any one of claims 8 to 10, wherein the magnetic surfactant is a ferrate-based ionic compound, in particular, wherein the magnetic surfactant is a ferrate-based ionic liquid.

12. The hand-held dispenser (300) of any one of claims 8 to 11, wherein the magnetic surfactant is selected from the group consisting of 1-methyl-trimethylimidazolium tetrachloroferrate, dodecyltrimethylammonium bromotrichloroferrate, or mixtures thereof.

13. The hand-held dispenser (300) of any one of claims 8 to 12, wherein the magnetic shaving aid composition comprises one or more of:.
- a humectant,
- an emulsifying agent,
- an emulsion stabilizer,
- a neutralizer,
- a skin conditioning agent,
- a propellant gas,
- a preservative,
- an oil or fatty compound,
- a non-magnetic surfactant,
- an emollient,
- a pH-adjusting agent,
- an antioxidant,
- chelating agents,
- a fragrant,
- a rheology modifier
- a gelling agent and
- a colorant
and, wherein the magnetic shaving aid composition further comprises at least:
- the humectant,
- the emulsifying agent or an emulsifying system,
- the one or more emulsion stabilizer, and
- the one or more skin conditioning agents.

14. The hand-held dispenser (300) of claim 13, wherein the magnetic shaving aid composition is in the form of a shaving cream, a shaving gel or a shaving foam, specifically in the form of a shaving gel or a shaving cream, in particular the magnetic shaving aid composition is dispensed in the form of a shaving cream and wherein the magnetic shaving aid composition comprises one or more preservatives, or,
wherein the magnetic shaving aid composition is dispensed in the form of a shaving gel and wherein the magnetic shaving aid composition comprises a propellant gas such as isopentane or isobutane in particular in an amount of 0.5-20 wt.-%, more specifically 1-10 wt.-%.

15. A method for avoiding skin irritation and improving the shave efficiency during a shaving operation by a user with a razor (100) in combination with a magnetic shaving aid comprising the steps of:
a) providing a razor (100) having a razor cartridge (20) and at least one magnetic element (10);
b) providing a magnetic shaving aid consisting of a magnetic shaving aid composition comprising a magnetic surfactant in a pre-shave state;
c) applying the magnetic shaving aid to a skin surface (K);
d) performing a shaving operation with the razor (100) on the skin surface (K);
e) inducing a change in the magnetic shaving aid by applying a magnetic field in the magnetic shaving aid to bring the magnetic shaving aid from the pre-shave state to a during-shave state during performing the shaving operation.
